(19) Europäisches Patentamt / European Patent Office / Office européen des brevets

(11) **EP 2 857 040 A1**

(12) **EUROPEAN PATENT APPLICATION**

(43) Date of publication:
08.04.2015 Bulletin 2015/15

(51) Int Cl.:
*A61K 39/395* (2006.01)  *G01N 33/574* (2006.01)

(21) Application number: 14193190.7

(22) Date of filing: 18.01.2011

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 19.01.2010 EP 10151109

(62) Document number(s) of the earlier application(s) in accordance with Art. 76 EPC:
11701059.5 / 2 525 821

(27) Previously filed application:
18.01.2011 PCT/EP2011/050564

(71) Applicant: F. Hoffmann-La Roche AG
4070 Basel (CH)

(72) Inventors:
• **Foernzler, Dorothee**
**5600 Lenzburg (CH)**
• **Delmar, Paul**
**4057 Basel (CH)**
• **Scherer, Stefan**
**South San Francisco, CA 94080 (US)**

(74) Representative: **Brodbeck, Michel**
**F. Hoffmann-La Roche AG**
**Patent Department**
**Grenzacherstrasse 124**
**4070 Basel (CH)**

Remarks:
This application was filed on 14-11-2014 as a divisional application to the application mentioned under INID code 62.

(54) **Tumor tissue based biomarkers for bevacizumab combination therapies**

(57) The present invention provides methods for improving the progression-free survival of a patient suffering from gastrointestinal cancer, in particular, metastatic colorectal cancer (mCRC), by treatment with bevacizumab (Avastin®) in combination with a chemotherapy regimen by determining the expression level of one or more of VEGFA and HER2 relative to control levels in patients diagnosed with gastrointestinal cancer, in particular, metastatic colorectal cancer (mCRC). The present invention further provides for methods for assessing the sensitivity or responsiveness of a patient to bevacizumab (Avastin®) in combination with a chemotherapy regimen, by determining the expression level of one or more of VEGFA and HER2 relative to control levels in patients diagnosed with gastrointestinal cancer, in particular, metastatic colorectal cancer (mCRC).

Figure 1.

EP 2 857 040 A1

Printed by Jouve, 75001 PARIS (FR)

SEQUENCE LISTING

<110> F. Hoffmann-La Roche Ltd

<120> Tumor tissue based biomarkers for bevacizumab combination therapies

<130> Case 26192

<150> EP 10151109.5
<151> 2010-01-19

<160> 3

<170> PatentIn version 3.5

<210> 1
<211> 232
<212> PRT
<213> Homo sapiens

<400> 1

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
            85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
            115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Tyr
145                 150                 155                 160

Lys Ser Trp Ser Val Tyr Val Gly Ala Arg Cys Cys Leu Met Pro Trp
                165                 170                 175

Ser Leu Pro Gly Pro His Pro Cys Gly Pro Cys Ser Glu Arg Arg Lys

His Leu Phe Val Gln Asp Pro Gln Thr Cys Lys Cys Ser Cys Lys Asn
195 200 205

Thr Asp Ser Arg Cys Lys Ala Arg Gln Leu Glu Leu Asn Glu Arg Thr
210 215 220

Cys Arg Cys Asp Lys Pro Arg Arg
225 230

<210> 2
<211> 1255
<212> PRT
<213> Homo sapiens

<400> 2

Met Glu Leu Ala Ala Leu Cys Arg Trp Gly Leu Leu Leu Ala Leu Leu
1 5 10 15

Pro Pro Gly Ala Ala Ser Thr Gln Val Cys Thr Gly Thr Asp Met Lys
20 25 30

Leu Arg Leu Pro Ala Ser Pro Glu Thr His Leu Asp Met Leu Arg His
35 40 45

Leu Tyr Gln Gly Cys Gln Val Val Gln Gly Asn Leu Glu Leu Thr Tyr
50 55 60

Leu Pro Thr Asn Ala Ser Leu Ser Phe Leu Gln Asp Ile Gln Glu Val
65 70 75 80

Gln Gly Tyr Val Leu Ile Ala His Asn Gln Val Arg Gln Val Pro Leu
85 90 95

Gln Arg Leu Arg Ile Val Arg Gly Thr Gln Leu Phe Glu Asp Asn Tyr
100 105 110

Ala Leu Ala Val Leu Asp Asn Gly Asp Pro Leu Asn Asn Thr Thr Pro
115 120 125

Val Thr Gly Ala Ser Pro Gly Gly Leu Arg Glu Leu Gln Leu Arg Ser
130 135 140

Leu Thr Glu Ile Leu Lys Gly Gly Val Leu Ile Gln Arg Asn Pro Gln
145 150 155 160

Leu Cys Tyr Gln Asp Thr Ile Leu Trp Lys Asp Ile Phe His Lys Asn
165 170 175

Asn Gln Leu Ala Leu Thr Leu Ile Asp Thr Asn Arg Ser Arg Ala Cys
180 185 190

His Pro Cys Ser Pro Met Cys Lys Gly Ser Arg Cys Trp Gly Glu Ser
        195                 200                 205

Ser Glu Asp Cys Gln Ser Leu Thr Arg Thr Val Cys Ala Gly Gly Cys
    210                 215                 220

Ala Arg Cys Lys Gly Pro Leu Pro Thr Asp Cys Cys His Glu Gln Cys
225                 230                 235                 240

Ala Ala Gly Cys Thr Gly Pro Lys His Ser Asp Cys Leu Ala Cys Leu
            245                 250                 255

His Phe Asn His Ser Gly Ile Cys Glu Leu His Cys Pro Ala Leu Val
            260                 265                 270

Thr Tyr Asn Thr Asp Thr Phe Glu Ser Met Pro Asn Pro Glu Gly Arg
        275                 280                 285

Tyr Thr Phe Gly Ala Ser Cys Val Thr Ala Cys Pro Tyr Asn Tyr Leu
    290                 295                 300

Ser Thr Asp Val Gly Ser Cys Thr Leu Val Cys Pro Leu His Asn Gln
305                 310                 315                 320

Glu Val Thr Ala Glu Asp Gly Thr Gln Arg Cys Glu Lys Cys Ser Lys
            325                 330                 335

Pro Cys Ala Arg Val Cys Tyr Gly Leu Gly Met Glu His Leu Arg Glu
            340                 345                 350

Val Arg Ala Val Thr Ser Ala Asn Ile Gln Glu Phe Ala Gly Cys Lys
        355                 360                 365

Lys Ile Phe Gly Ser Leu Ala Phe Leu Pro Glu Ser Phe Asp Gly Asp
    370                 375                 380

Pro Ala Ser Asn Thr Ala Pro Leu Gln Pro Glu Gln Leu Gln Val Phe
385                 390                 395                 400

Glu Thr Leu Glu Glu Ile Thr Gly Tyr Leu Tyr Ile Ser Ala Trp Pro
            405                 410                 415

Asp Ser Leu Pro Asp Leu Ser Val Phe Gln Asn Leu Gln Val Ile Arg
            420                 425                 430

Gly Arg Ile Leu His Asn Gly Ala Tyr Ser Leu Thr Leu Gln Gly Leu
        435                 440                 445

Gly Ile Ser Trp Leu Gly Leu Arg Ser Leu Arg Glu Leu Gly Ser Gly
    450                 455                 460

Leu Ala Leu Ile His His Asn Thr His Leu Cys Phe Val His Thr Val
465                 470                 475                 480

Pro Trp Asp Gln Leu Phe Arg Asn Pro His Gln Ala Leu Leu His Thr
                485                 490                 495

Ala Asn Arg Pro Glu Asp Glu Cys Val Gly Glu Gly Leu Ala Cys His
            500                 505                 510

Gln Leu Cys Ala Arg Gly His Cys Trp Gly Pro Gly Pro Thr Gln Cys
        515                 520                 525

Val Asn Cys Ser Gln Phe Leu Arg Gly Gln Glu Cys Val Glu Glu Cys
    530                 535                 540

Arg Val Leu Gln Gly Leu Pro Arg Glu Tyr Val Asn Ala Arg His Cys
545                 550                 555                 560

Leu Pro Cys His Pro Glu Cys Gln Pro Gln Asn Gly Ser Val Thr Cys
            565                 570                 575

Phe Gly Pro Glu Ala Asp Gln Cys Val Ala Cys Ala His Tyr Lys Asp
            580                 585                 590

Pro Pro Phe Cys Val Ala Arg Cys Pro Ser Gly Val Lys Pro Asp Leu
        595                 600                 605

Ser Tyr Met Pro Ile Trp Lys Phe Pro Asp Glu Glu Gly Ala Cys Gln
    610                 615                 620

Pro Cys Pro Ile Asn Cys Thr His Ser Cys Val Asp Leu Asp Asp Lys
625                 630                 635                 640

Gly Cys Pro Ala Glu Gln Arg Ala Ser Pro Leu Thr Ser Ile Ile Ser
            645                 650                 655

Ala Val Val Gly Ile Leu Leu Val Val Val Leu Gly Val Val Phe Gly
            660                 665                 670

Ile Leu Ile Lys Arg Arg Gln Gln Lys Ile Arg Lys Tyr Thr Met Arg
        675                 680                 685

Arg Leu Leu Gln Glu Thr Glu Leu Val Glu Pro Leu Thr Pro Ser Gly
    690                 695                 700

Ala Met Pro Asn Gln Ala Gln Met Arg Ile Leu Lys Glu Thr Glu Leu
705                 710                 715                 720

Arg Lys Val Lys Val Leu Gly Ser Gly Ala Phe Gly Thr Val Tyr Lys
            725                 730                 735

Gly Ile Trp Ile Pro Asp Gly Glu Asn Val Lys Ile Pro Val Ala Ile
        740                 745                 750

Lys Val Leu Arg Glu Asn Thr Ser Pro Lys Ala Asn Lys Glu Ile Leu
        755                 760                 765

Asp Glu Ala Tyr Val Met Ala Gly Val Gly Ser Pro Tyr Val Ser Arg
    770                 775                 780

Leu Leu Gly Ile Cys Leu Thr Ser Thr Val Gln Leu Val Thr Gln Leu
785                 790                 795                 800

Met Pro Tyr Gly Cys Leu Leu Asp His Val Arg Glu Asn Arg Gly Arg
            805                 810                 815

Leu Gly Ser Gln Asp Leu Leu Asn Trp Cys Met Gln Ile Ala Lys Gly
            820                 825                 830

Met Ser Tyr Leu Glu Asp Val Arg Leu Val His Arg Asp Leu Ala Ala
        835                 840                 845

Arg Asn Val Leu Val Lys Ser Pro Asn His Val Lys Ile Thr Asp Phe
    850                 855                 860

Gly Leu Ala Arg Leu Leu Asp Ile Asp Glu Thr Glu Tyr His Ala Asp
865                 870                 875                 880

Gly Gly Lys Val Pro Ile Lys Trp Met Ala Leu Glu Ser Ile Leu Arg
                885                 890                 895

Arg Arg Phe Thr His Gln Ser Asp Val Trp Ser Tyr Gly Val Thr Val
            900                 905                 910

Trp Glu Leu Met Thr Phe Gly Ala Lys Pro Tyr Asp Gly Ile Pro Ala
        915                 920                 925

Arg Glu Ile Pro Asp Leu Leu Glu Lys Gly Glu Arg Leu Pro Gln Pro
    930                 935                 940

Pro Ile Cys Thr Ile Asp Val Tyr Met Ile Met Val Lys Cys Trp Met
945                 950                 955                 960

Ile Asp Ser Glu Cys Arg Pro Arg Phe Arg Glu Leu Val Ser Glu Phe
                965                 970                 975

Ser Arg Met Ala Arg Asp Pro Gln Arg Phe Val Val Ile Gln Asn Glu
            980                 985                 990

Asp Leu Gly Pro Ala Ser Pro Leu  Asp Ser Thr Phe Tyr  Arg Ser Leu
        995                 1000                1005

```
Leu Glu Asp Asp Asp Met Gly Asp Leu Val Asp Ala Glu Glu Tyr
    1010            1015            1020

Leu Val Pro Gln Gln Gly Phe Phe Cys Pro Asp Pro Ala Pro Gly
    1025            1030            1035

Ala Gly Gly Met Val His His Arg His Arg Ser Ser Ser Thr Arg
    1040            1045            1050

Ser Gly Gly Gly Asp Leu Thr Leu Gly Leu Glu Pro Ser Glu Glu
    1055            1060            1065

Glu Ala Pro Arg Ser Pro Leu Ala Pro Ser Glu Gly Ala Gly Ser
    1070            1075            1080

Asp Val Phe Asp Gly Asp Leu Gly Met Gly Ala Ala Lys Gly Leu
    1085            1090            1095

Gln Ser Leu Pro Thr His Asp Pro Ser Pro Leu Gln Arg Tyr Ser
    1100            1105            1110

Glu Asp Pro Thr Val Pro Leu Pro Ser Glu Thr Asp Gly Tyr Val
    1115            1120            1125

Ala Pro Leu Thr Cys Ser Pro Gln Pro Glu Tyr Val Asn Gln Pro
    1130            1135            1140

Asp Val Arg Pro Gln Pro Pro Ser Pro Arg Glu Gly Pro Leu Pro
    1145            1150            1155

Ala Ala Arg Pro Ala Gly Ala Thr Leu Glu Arg Pro Lys Thr Leu
    1160            1165            1170

Ser Pro Gly Lys Asn Gly Val Val Lys Asp Val Phe Ala Phe Gly
    1175            1180            1185

Gly Ala Val Glu Asn Pro Glu Tyr Leu Thr Pro Gln Gly Gly Ala
    1190            1195            1200

Ala Pro Gln Pro His Pro Pro Pro Ala Phe Ser Pro Ala Phe Asp
    1205            1210            1215

Asn Leu Tyr Tyr Trp Asp Gln Asp Pro Pro Glu Arg Gly Ala Pro
    1220            1225            1230

Pro Ser Thr Phe Lys Gly Thr Pro Thr Ala Glu Asn Pro Glu Tyr
    1235            1240            1245

Leu Gly Leu Asp Val Pro Val
    1250            1255
```

20

```
<210>   3
<211>   923
<212>   PRT
<213>   Homo sapiens

<400>   3
```

Met Glu Arg Gly Leu Pro Leu Leu Cys Ala Val Leu Ala Leu Val Leu
1               5                   10                  15

Ala Pro Ala Gly Ala Phe Arg Asn Asp Lys Cys Gly Asp Thr Ile Lys
            20                  25                  30

Ile Glu Ser Pro Gly Tyr Leu Thr Ser Pro Gly Tyr Pro His Ser Tyr
        35                  40                  45

His Pro Ser Glu Lys Cys Glu Trp Leu Ile Gln Ala Pro Asp Pro Tyr
        50                  55                  60

Gln Arg Ile Met Ile Asn Phe Asn Pro His Phe Asp Leu Glu Asp Arg
65                  70                  75                  80

Asp Cys Lys Tyr Asp Tyr Val Glu Val Phe Asp Gly Glu Asn Glu Asn
                85                  90                  95

Gly His Phe Arg Gly Lys Phe Cys Gly Lys Ile Ala Pro Pro Pro Val
            100                 105                 110

Val Ser Ser Gly Pro Phe Leu Phe Ile Lys Phe Val Ser Asp Tyr Glu
            115                 120                 125

Thr His Gly Ala Gly Phe Ser Ile Arg Tyr Glu Ile Phe Lys Arg Gly
        130                 135                 140

Pro Glu Cys Ser Gln Asn Tyr Thr Thr Pro Ser Gly Val Ile Lys Ser
145                 150                 155                 160

Pro Gly Phe Pro Glu Lys Tyr Pro Asn Ser Leu Glu Cys Thr Tyr Ile
                165                 170                 175

Val Phe Val Pro Lys Met Ser Glu Ile Ile Leu Glu Phe Glu Ser Phe
            180                 185                 190

Asp Leu Glu Pro Asp Ser Asn Pro Pro Gly Gly Met Phe Cys Arg Tyr
            195                 200                 205

Asp Arg Leu Glu Ile Trp Asp Gly Phe Pro Asp Val Gly Pro His Ile
        210                 215                 220

Gly Arg Tyr Cys Gly Gln Lys Thr Pro Gly Arg Ile Arg Ser Ser Ser
225                 230                 235                 240

Gly Ile Leu Ser Met Val Phe Tyr Thr Asp Ser Ala Ile Ala Lys Glu
                245             250             255

Gly Phe Ser Ala Asn Tyr Ser Val Leu Gln Ser Ser Val Ser Glu Asp
            260             265             270

Phe Lys Cys Met Glu Ala Leu Gly Met Glu Ser Gly Glu Ile His Ser
        275             280             285

Asp Gln Ile Thr Ala Ser Ser Gln Tyr Ser Thr Asn Trp Ser Ala Glu
    290             295             300

Arg Ser Arg Leu Asn Tyr Pro Glu Asn Gly Trp Thr Pro Gly Glu Asp
305             310             315             320

Ser Tyr Arg Glu Trp Ile Gln Val Asp Leu Gly Leu Leu Arg Phe Val
            325             330             335

Thr Ala Val Gly Thr Gln Gly Ala Ile Ser Lys Glu Thr Lys Lys Lys
            340             345             350

Tyr Tyr Val Lys Thr Tyr Lys Ile Asp Val Ser Ser Asn Gly Glu Asp
        355             360             365

Trp Ile Thr Ile Lys Glu Gly Asn Lys Pro Val Leu Phe Gln Gly Asn
    370             375             380

Thr Asn Pro Thr Asp Val Val Val Ala Val Phe Pro Lys Pro Leu Ile
385             390             395             400

Thr Arg Phe Val Arg Ile Lys Pro Ala Thr Trp Glu Thr Gly Ile Ser
            405             410             415

Met Arg Phe Glu Val Tyr Gly Cys Lys Ile Thr Asp Tyr Pro Cys Ser
            420             425             430

Gly Met Leu Gly Met Val Ser Gly Leu Ile Ser Asp Ser Gln Ile Thr
        435             440             445

Ser Ser Asn Gln Gly Asp Arg Asn Trp Met Pro Glu Asn Ile Arg Leu
    450             455             460

Val Thr Ser Arg Ser Gly Trp Ala Leu Pro Pro Ala Pro His Ser Tyr
465             470             475             480

Ile Asn Glu Trp Leu Gln Ile Asp Leu Gly Glu Glu Lys Ile Val Arg
            485             490             495

Gly Ile Ile Ile Gln Gly Gly Lys His Arg Glu Asn Lys Val Phe Met
        500             505             510

Arg Lys Phe Lys Ile Gly Tyr Ser Asn Asn Gly Ser Asp Trp Lys Met
        515                 520                 525

Ile Met Asp Asp Ser Lys Arg Lys Ala Lys Ser Phe Glu Gly Asn Asn
        530                 535                 540

Asn Tyr Asp Thr Pro Glu Leu Arg Thr Phe Pro Ala Leu Ser Thr Arg
545                 550                 555                 560

Phe Ile Arg Ile Tyr Pro Glu Arg Ala Thr His Gly Gly Leu Gly Leu
                565                 570                 575

Arg Met Glu Leu Leu Gly Cys Glu Val Glu Ala Pro Thr Ala Gly Pro
            580                 585                 590

Thr Thr Pro Asn Gly Asn Leu Val Asp Glu Cys Asp Asp Asp Gln Ala
        595                 600                 605

Asn Cys His Ser Gly Thr Gly Asp Asp Phe Gln Leu Thr Gly Gly Thr
    610                 615                 620

Thr Val Leu Ala Thr Glu Lys Pro Thr Val Ile Asp Ser Thr Ile Gln
625                 630                 635                 640

Ser Glu Phe Pro Thr Tyr Gly Phe Asn Cys Glu Phe Gly Trp Gly Ser
                645                 650                 655

His Lys Thr Phe Cys His Trp Glu His Asp Asn His Val Gln Leu Lys
            660                 665                 670

Trp Ser Val Leu Thr Ser Lys Thr Gly Pro Ile Gln Asp His Thr Gly
            675                 680                 685

Asp Gly Asn Phe Ile Tyr Ser Gln Ala Asp Glu Asn Gln Lys Gly Lys
    690                 695                 700

Val Ala Arg Leu Val Ser Pro Val Val Tyr Ser Gln Asn Ser Ala His
705                 710                 715                 720

Cys Met Thr Phe Trp Tyr His Met Ser Gly Ser His Val Gly Thr Leu
                725                 730                 735

Arg Val Lys Leu Arg Tyr Gln Lys Pro Glu Glu Tyr Asp Gln Leu Val
            740                 745                 750

Trp Met Ala Ile Gly His Gln Gly Asp His Trp Lys Glu Gly Arg Val
        755                 760                 765

Leu Leu His Lys Ser Leu Lys Leu Tyr Gln Val Ile Phe Glu Gly Glu
    770                 775                 780

```
Ile Gly Lys Gly Asn Leu Gly Gly Ile Ala Val Asp Asp Ile Ser Ile
785             790             795                         800

Asn Asn His Ile Ser Gln Glu Asp Cys Ala Lys Pro Ala Asp Leu Asp
            805             810                     815

Lys Lys Asn Pro Glu Ile Lys Ile Asp Glu Thr Gly Ser Thr Pro Gly
            820             825             830

Tyr Glu Gly Glu Gly Glu Gly Asp Lys Asn Ile Ser Arg Lys Pro Gly
        835             840             845

Asn Val Leu Lys Thr Leu Asp Pro Ile Leu Ile Thr Ile Ile Ala Met
    850             855             860

Ser Ala Leu Gly Val Leu Leu Gly Ala Val Cys Gly Val Val Leu Tyr
865             870             875                         880

Cys Ala Cys Trp His Asn Gly Met Ser Glu Arg Asn Leu Ser Ala Leu
            885             890                     895

Glu Asn Tyr Asn Phe Glu Leu Val Asp Gly Val Lys Leu Lys Lys Asp
            900             905             910

Lys Leu Asn Thr Gln Ser Thr Tyr Ser Glu Ala
            915             920
```

**Description**

The present invention provides methods for improving the progression-free survival of a patient suffering from gastrointestinal cancer, in particular, metastatic colorectal cancer (mCRC), by treatment with bevacizumab (Avastin®) in combination with a chemotherapy regimen by determining the expression level of one or more of VEGFA, HER2 and neuropilin relative to control levels in patients diagnosed with gastrointestinal cancer, in particular, metastatic colorectal cancer (mCRC). The present invention further provides for methods for assessing the sensitivity or responsiveness of a patient to bevacizumab (Avastin®) in combination with a chemotherapy regimen, by determining the expression level of one or more of VEGFA, HER2 and neuropilin relative to control levels in patients diagnosed with gastrointestinal cancer, in particular, metastatic colorectal cancer (mCRC).

Accordingly, the present invention relates to the identification and selection of biomarkers of gastrointestinal cancers, in particular, of metastatic colorectal cancer (mCRC), that correlate with sensitivity or responsiveness to angiogenesis inhibitors, *e.g.,* bevacizumab (Avastin®), in combination with chemotherapeutic regimens, such as oxaliplatin-based chemotherapies. In this respect, the invention relates to the use of (a) tumor specific expression profile(s) of one or more of VEGFA, HER2 and neuropilin, relative to controls established in patients diagnosed with gastrointestinal cancer, in particular, mCRC, to identify patients sensitive or responsive to the addition of angiogenesis inhibitors, *e.g.,* bevacizumab (Avastin®), to standard chemotherapies. The invention further relates to methods for improving progression-free survival of a patient suffering from gastrointestinal cancer, in particular, mCRC, by the addition of angiogenesis inhibitors, *e.g.,* bevacizumab (Avastin®), to standard chemotherapies, *e.g.,* oxaliplatin-based chemotherapies, by determining (a) tumor specific expression level(s) of one or more of VEGFA, HER2 and neuropilin relative to control(s) in patients diagnosed with gastrointestinal cancer, in particular, metastatic colorectal cancer. As an alternative or in addition to the determination of the expression level of one or more of VEGFA, HER2 and neuropilin according to the methods described herein, the vessel number in a tumor sample, relative to (a) control level(s) established in patients diagnosed with gastrointestinal cancer, in particular, mCRC, can be determined as a biomarker as an indicator of a patient sensitive or responsive to the addition of angiogenesis inhibitors, *e.g.,* bevacizumab (Avastin®), to standard chemotherapies. The invention also provides for kits and compositions for identification of patients sensitive or responsive to angiogenesis inhibitors, in particular, bevacizumab (Avastin®), determined and defined in accordance with the methods of the present invention.

Angiogenesis is necessary for cancer development, regulating not only primary tumor size and growth but also impacting invasive and metastatic potential. Accordingly, the mechanisms mediating angiogenic processes have been investigated as potential targets for directed anti-cancer therapies. Early in the study of angiogenic modulators, the vascular endothelial growth factor (VEGF) signalling pathway was discovered to preferentially regulate angiogenic activity in multiple cancer types and multiple therapeutics have been developed to modulate this pathway at various points. These therapies include, among others, bevacizumab, sunitinib, sorafenib and vatalanib. Although the use of angiogenic inhibitors in the clinic has shown success, not all patients respond or fail to fully respond to angiogenesis inhibitor therapy. The mechanism(s) underlying such incomplete response is unknown. Therefore, there is an increasing need for the identification of patient subgroups sensitive or responsive to anti-angiogenic cancer therapy.

While a number of angiogenesis inhibitors are known, the most prominent angiogenesis inhibitor is Bevacizumab (Avastin®). Bevacizumab is a recombinant humanized monoclonal IgG1 antibody that specifically binds and blocks the biological effects of VEGF (vascular endothelial growth factor). VEGF is a key driver of tumor angiogenesis - an essential process required for tumor growth and metastasis, *i.e.,* the dissemination of the tumor to other parts of the body. Avastin® has been approved in Europe for the treatment of the advanced stages of four common types of cancer: colorectal cancer, breast cancer, non-small cell lung cancer (NSCLC) and kidney cancer, which collectively cause over 2.5 million deaths each year. In the United States, Avastin® was the first anti-angiogenesis therapy approved by the FDA, and it is now approved for the treatment of five tumor types: colorectal cancer, non-small cell lung cancer, breast cancer, brain (glioblastoma) and kidney (renal cell carcinoma). Over half a million patients have been treated with Avastin so far, and a comprehensive clinical program with over 450 clinical trials is investigating the further use of Avastin in the treatment of multiple cancer types (including colorectal, breast, non-small cell lung, brain, gastric, ovarian and prostate) in different settings (*e.g.,* advanced or early stage disease). Importantly, Avastin® has shown promise as a co-therapeutic, demonstrating efficacy when combined with a broad range of chemotherapies and other anti-cancer treatments. Phase-III studies have been published demonstrating the beneficial effects of combining bevacizumab with standard chemotherapeutic regimens (see, *e.g.,* Saltz et al., 2008, J. Clin. Oncol., 26:2013-2019; Yang et al., 2008, Clin. Cancer Res., 14:5893-5899; Hurwitz et al., 2004, N. Engl. J. Med., 350:2335-2342). However, as in previous studies of angiogenic inhibitors, some of these phase-III studies have shown that a portion of patients experience incomplete response to the addition of bevacizumab (Avastin®) to their chemotherapeutic regimens.

Accordingly, there is a need for methods of determining those patients that respond or are likely to respond to combination therapies comprising angiogenesis inhibitors, in particular, bevacizumab (Avastin®). Thus, the technical problem underlying the present invention is the provision of methods and means for the identification of (a) patient(s) suffering from or prone to suffer from gastrointestinal cancer, in particular mCRC, who may benefit from the addition of angiogenesis inhibitors, in particular, bevacizumab (Avastin®), to chemotherapeutic regimens, *e.g.,* oxaliplatin-based inhibitors.

The technical problem is solved by provision of the embodiments characterized in the claims.

The present invention, therefore, provides a method for improving the treatment effect of a chemotherapy regimen of a patient suffering from gastrointestinal cancer, in particular, mCRC, by adding bevacizumab to said chemotherapy regimen, said method comprising:

(a) determining the expression level of one or more of VEGFA, HER2 and neuropilin; and
(b) administering bevacizumab in combination with a chemotherapy regimen to the patient having an increased level of VEGFA, and/or a decreased level of HER2 and/or neuropilin relative to control levels determined in patients diagnosed with gastrointestinal cancer, in particular, mCRC.

The present invention relates to a method for improving the treatment effect of a chemotherapy regimen of a patient suffering from gastrointestinal cancer, in particular, mCRC, by adding bevacizumab to the chemotherapy regimen, said method comprising:

(a) obtaining a sample from said patient;
(b) determining the expression level of one or more of VEGFA, HER2 and neuropilin; and
(c) administering bevacizumab in combination with a chemotherapy regimen to the patient having an increased level of VEGFA, and/or a decreased level of HER2 and/or neuropilin relative to control levels determined in patients diagnosed with gastrointestinal cancer, in particular, mCRC.

The present invention, therefore, provides a method for improving the progression-free survival of a patient suffering from gastrointestinal cancer, in particular, mCRC, by adding bevacizumab to a chemotherapy regimen, said method comprising:

(a) determining the expression level of one or more of VEGFA, HER2 and neuropilin; and
(b) administering bevacizumab in combination with a chemotherapy regimen to the patient having an increased level of VEGFA, and/or a decreased level of HER2 and/or neuropilin relative to control levels determined in patients diagnosed with gastrointestinal cancer, in particular, mCRC.

The present invention, therefore, provides a method for improving the progression-free survival of a patient suffering from gastrointestinal cancer, in particular, mCRC, by adding bevacizumab to a chemotherapy regimen, said method comprising:

(a) obtaining a sample from said patient;
(b) determining the expression level of one or more of VEGFA, HER2 and neuropilin; and
(c) administering bevacizumab in combination with a chemotherapy regimen to the patient having an increased level of VEGFA, and/or a decreased level of HER2 and/or neuropilin relative to control levels determined in patients diagnosed with gastrointestinal cancer, in particular, mCRC.

In an alternative embodiment, the present invention relates to an in vitro method for the identification of a patient responsive to or sensitive to the addition of bevacizumab to a chemotherapy regimen, said method comprising:

(a) obtaining a sample from a patient suspected to suffer or being prone to suffer from gastrointestinal cancer, in particular, mCRC; and
(b) determining the expression level of one or more of VEGFA, HER2 and neuropilin;

whereby an increased level of VEGFA, and/or a decreased level of HER2 and/or neuropilin relative to control levels determined in patients diagnosed with gastrointestinal cancer, in particular, mCRC, is indicative of a sensitivity of the patient to the addition of bevacizumab to said regimen.

As an alternative or in addition to the determination of the expression level of one or more of VEGFA, HER2 and neuropilin according to the methods described herein, the vessel number in a tumor sample, relative to (a) control level(s) established in patients diagnosed with gastrointestinal cancer, in particular, mCRC, can be determined as a biomarker as an indicator of a patient sensitive or responsive to the addition of angiogenesis inhibitors, e.g., bevacizumab (Avastin®), to standard chemotherapies. Therefore, methods of the present invention encompass the determination of the vessel number in said sample where such vessel number determination is possible or expected to be possible as recognized by the skilled artisan, *e.g.,* in solid tissue samples such as tissue biopsies and/or tissue resections. Vessel number determination may be performed by any method described herein or as known in the art for such measurement. An exemplary method for vessel number determination is the detection of markers for endothelial cells by using one or more antibodies specific for one or more endothelial cell markers. In preferred embodiments, the biomarker for the endothelial cells is not expressed by the tumor cells. Because the vessel structure is formed from endothelial cells, the one or more endothelial cell markers distinguish vessel structure from tumor (cells), allowing vessel number to be readily determined. The skilled artisan, *e.g.,* a pathologist, will be able to readily determine both suitable antibodies for detection/distinguishing endothelial cells (in particular, relative to tumor cells) as well as methods for detection of such antibodies and subsequent analysis of the sample. The analysis of the sample according to the methods of the invention may be manual, as performed by the skilled artisan, *e.g.,* a pathologist, as is known in the art, or may be automated using commercially available software designed for the processing and analysis of pathology images, *e.g.,* for determination of vessel number or other analysis in tissue biopsies or resections (*e.g.,* MIRAX SCAN, Carl Zeiss AG, Jena, Germany).

An exemplary antigen recognized as an endothelial cell marker for use in determination of vessel number according to the methods of the invention is CD31. The antigen CD31 is recognized, for example, by antibody clone JC70A available from Dako A/S (Glostrup, Denmark) under product number M0823, the use thereof is encompassed by the methods of the invention. Accordingly, the invention encompasses the determination of the tumor specific expression level or expression pattern of CD31 in a patient sample (1) as an indicator of sensitivity or responsiveness of said patient to the addition of bevacizumab to a chemotherapeutic regimen in said patient, or (2) as part of a method to improve the progression free survival of said patient, wherein the patient suffers from, or is expected to suffer from, gastrointestinal cancer, in particular, mCRC. Because CD31 stains endothelial cells, and greater vessel number correlates with a greater endothelial cell number, the vessel number in a sample is also directly correlated to the tumor specific CD31 expression level. Accordingly the invention encompasses methods for improving the progression-free survival of a patient suffering from gastrointestinal cancer comprising determining the tumor specific vessel number and/or tumor specific CD31 expression level in said patient and administering bevacizumab in combination with a chemotherapy regimen to the patient having an increased vessel number (and/or increased CD31 expression) relative to control levels determined in patients diagnosed with gastrointestinal cancer, in particular, mCRC. Similarly, the invention encompasses an in vitro method for the identification of a patient responsive to or sensitive to the addition of bevacizumab to a chemotherapy regimen comprising determining the tumor specific vessel number and/or tumor specific CD31 expression level in said patient and whereby an increased vessel number (and/or increased CD31 expression) in a tumor sample from said patient relative to control levels determined in patients diagnosed with gastrointestinal cancer, in particular, mCRC, is indicative of a sensitivity of the patient to the addition of bevacizumab to said regimen.

Accordingly, the present invention solves the identified technical problem in that it was surprisingly shown that the tumor specific expression levels of one or more of VEGFA, HER2 and neuropilin in a given patient, relative to control levels determined in patients diagnosed gastrointestinal cancer, in particular, mCRC, correlate with treatment effect in those patients administered an angiogenesis inhibitor in combination with a chemotherapy regimen. Specifically, variations in the tumor specific expression levels of VEGFA, HER2 and/or neuropilin were surprisingly identified as markers/predictors for the improved progression-free survival of gastrointestinal cancer patients in response to the addition of bevacizumab (Avastin®) to oxaliplatin-based chemotherapeutic regimens. Patients exhibiting a response or sensitivity to the addition of bevacizumab (Avastin®) to chemotherapy regimens were identified to have one or more of increased expression of VEGFA, decreased expression of neuropilin and decreased expression of HER2 relative to control levels established in samples obtained from patients diagnosed with metastatic gastrointestinal cancer. Further, in addition to the altered expression of one or more of VEGFA, HER2 and/or neuropilin as herein described, increases in the tumor specific vessel number for a given patient (which correlates with the tumor specific expression level of one or more endothelial cell markers, e.g., CD31), relative to control levels established in patients diagnosed with gastrointestinal cancer, in particular, mCRC, were surprisingly identified (1) as one of the markers/predictors for the improved progression-free survival, and/or (2) as one of the markers/predictors that correlate with treatment effect in gastrointestinal cancer patients administered an angiogenesis inhibitor in combination with a chemotherapy regimen. The terms "marker" and "predictor" can be used interchangeably and refer to the expression levels of one or more of VEGFA, HER2 and neuropilin as described herein. In addition to the expression level of one or more of VEGFA, HER2 and/or neuropilin, the invention also encompasses the use of the terms "marker" and "predictor" to refer to the tumor specific vessel number and/or tumor specific expression level of an endothelial cell marker, *e.g.,* CD31, according to the methods described herein. The invention also encompasses the use of the terms "marker" and "predictor" to refer to a combination of any two or more of the tumor specific expression level of VEGFA, HER2 and neuropilin, and the tumor specific vessel number.

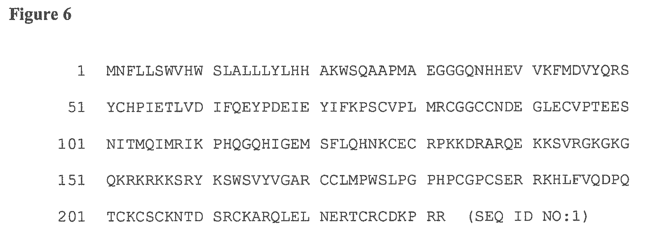

In the context of the present invention, "VEGFA" refers to vascular endothelial growth factor protein A, exemplified by SEQ ID NO:1, shown in FIGURE 6. The term "VEGFA" encompasses the protein having the amino acid sequence of SEQ ID NO:1 as well as homologues and isoforms thereof. The term "VEGFA" also encompasses the known isoforms, *e.g.,* splice isoforms, of VEGFA, *e.g.,* $VEGF_{121}$, $VEGF_{145}$, $VEGF_{165}$, $VEGF_{189}$ and $VEGF_{206}$, as well as variants, homologues and isoforms thereof In the context of the invention, the term "VEGFA" also encompasses proteins having at least 85%, at least 90% or at least 95% homology to the amino acid sequence of SEQ ID NO:1, or to the amino acid sequences of the variants and/or homologues thereof, as well as fragments of the sequences, provided that the variant proteins (including isoforms), homologous proteins and/or fragments are recognized by one or more VEGFA specific antibodies, such as antibody clone SP28 available from Abcam, Inc (Cambridge, Massachusetts, U.S.A.).

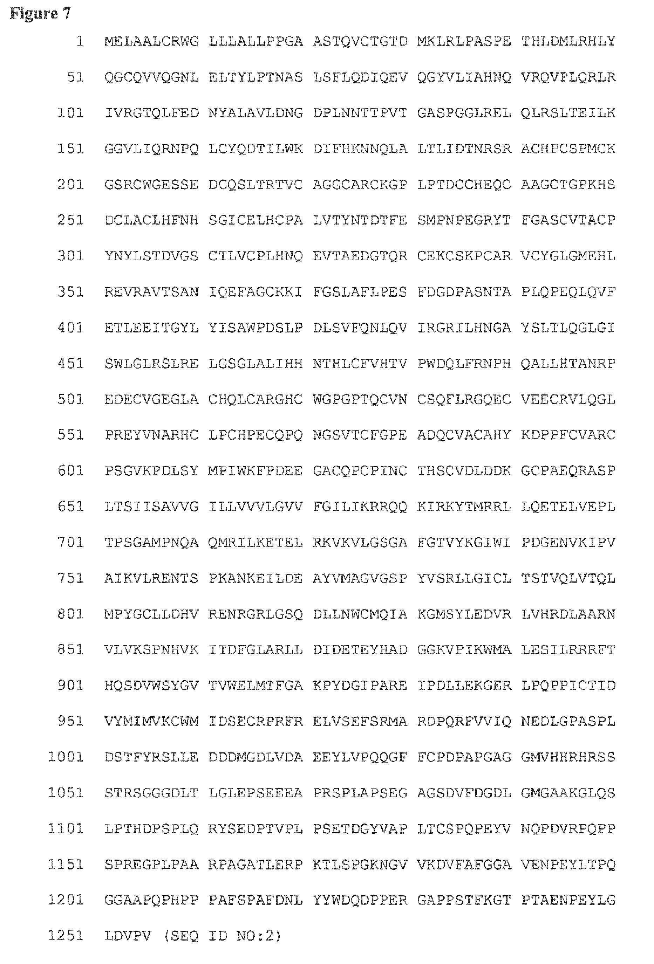

In the context of the present invention, "HER2" references the type I transmembrane protein, also known as c-erbB2, ErbB2 or Neu, belonging to the family of epidermal growth factor receptors, exemplified by the amino acid sequence SEQ ID NO:2, shown in FIGURE 7. In the context of the present invention, the term "HER2" also encompasses homologues, variants and isoforms, including splice isoforms, of HER2. The term "HER2" further encompasses proteins having at least 85%, at least 90% or at least 95% homology to the amino acid sequence of SEQ ID NO:2, or to the sequence of one or more of a HER2 homologue, variant and isoform, as well as fragments of the sequences, provided that the variant proteins (including isoforms), homologous proteins and/or fragments are recognized by one or more HER2 specific antibodies, such as provided as Herceptest™ available from Dako A/S (Glostrup, Denmark). The HER2 specific antibody in said Herceptest™ is an affinity purified rabbit antibody directed against a synthetic C-terminal intracytoplasmic fragment of the human HER2 protein (immunogen coupled to keyhole limpet hemocyanin). Further exemplary anti-HER2 antibodies that are commercially available and suitable for use according to the methods of the invention include, but are not limited to, clone 4B5 available from Ventana Medical Systems S.A. (Illkirch, France); one or more of clones CB11, 5A2, 10A7, and CBE1 available from Novocastra/Leica GmbH (Wetzler, Germany); clone SP3 available from Thermo Fisher Scientific (Fremont, CA, USA) and clone TAB250 available from Invitrogen™ (Carslbad, CA, USA).

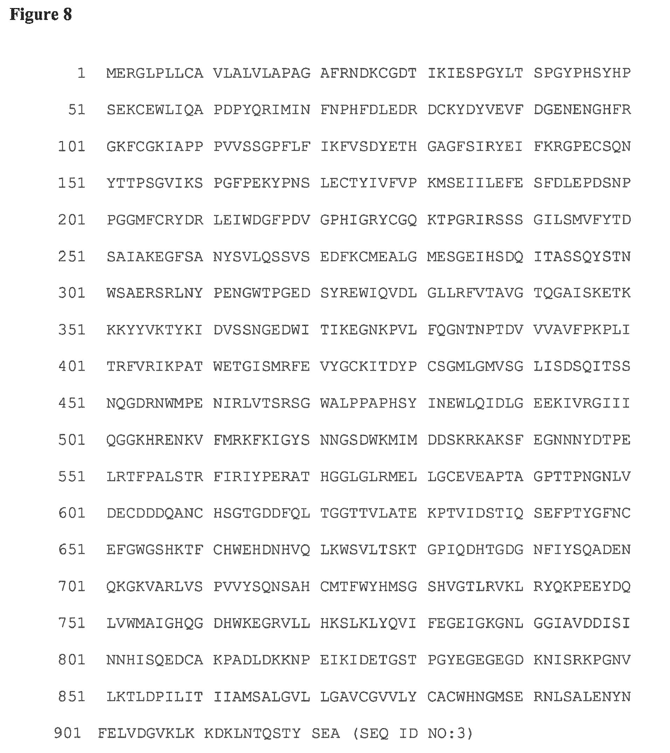

In the context of the present invention, "neuropilin" refers to the neuropilin-1 protein, a type-I membrane protein also known as NRP-1, and exemplified by the amino acid sequence SEQ ID NO:3, shown in FIGURE 8. As used herein, "neuropilin" may also refer to neuropilin-2/NRP-2, which shares approximately 44% homology to NRP-1 as known in the art. In the context of the present invention, the term "neuropilin" also encompasses homologs, variants and isoforms of NRP-1 and/or NRP-2. The term, "neuropilin" further encompasses proteins having at least 85%, at least 90% or at least 95% homology to the amino acid sequence of SEQ ID NO:1, or to the sequence of one or more of a NRP-1 and/or NRP-2 homologue, variant and isoform, including splice isoforms, as well as fragments of the sequences, provided that the variant proteins (including isoforms), homologous proteins and/or fragments are recognized by one or more NRP-1 and/or NRP-2 specific antibodies, such as clone 446915 available from R&D Systems, Inc. (Minneapolis, Minnesota, U.S.A.).

As an alternative or in addition to the determination of the tumor specific expression level of one or more of VEGFA, HER2 and neuropilin, the invention also encompasses the determination of vessel number as one of the biomarkers for use in the methods described herein. As known in the art and described herein, vessel number within a patient sample, *e.g.,* sample comprising tumor tissue, may, for example, be assessed by immunohistochemical methods detecting one or more endothelial cell markers, *e.g.,* CD31. CD31 is recognized as an endothelial cell marker suitable for the determination of vessel number in tumor samples, and, is commonly probed using specific antibodies such as the anti-CD31 antibody available from Dako A/S (Glostrup, Denmark) as clone JC70A under product number M0823. As an alternative or in addition to the determination of the tumor specific expression level of one or more of VEGFA, HER2 and neuropilin, the invention further encompasses the use of Dako A/S antibody clone JC70A (product number M0823) for determination of vessel number, detection of endothelial cells, and/or detection of the expression level of an endothelial cell marker according to the methods described herein.

Accordingly, the present invention encompasses the determination of expression levels of proteins including, but not limited to, the amino acid sequences as described herein. In this context the invention encompasses the detection of homologues, variants and isoforms of one or more of VEGFA, HER2 and neuropilin; said isoforms or variants may, inter alia, comprise allelic variants or splice variants. Also envisaged is the detection of proteins that are homologous to one or more of VEGFA, HER2 and neuropilin as herein described, or a fragment thereof, *e.g.,* having at least 60%, 70%, 80%, 90%, 95%, 96%, 97%, 98% or 99% sequence identity to the amino acid sequence of SEQ ID NO:1, SEQ ID NO:2 or SEQ ID NO:3 or a fragment thereof. Alternatively or additionally, the present invention encompasses detection of the expression levels of proteins encoded by nucleic acid sequences, or fragments thereof, that are at least at least 60%, 70%, 80%, 90%, 95%, 96%, 97%, 98% or 99% identical to a nucleic acid sequence encoding SEQ ID NO:1, SEQ ID NO:2 or SEQ ID NO:3 or a fragment, variant or isoform thereof. In this context, the term "variant" means that the VEGFA, HER2, and/or neuropilin amino acid sequence, or the nucleic acid sequence encoding said amino acid sequence, differs from the distinct sequences identified by SEQ ID NOs:1, SEQ ID NO:2 or SEQ ID NO:3 and/or available under the above-identified GenBank Accession numbers, by mutations, *e.g.,* deletion, additions, substitutions, inversions etc. In addition, the term "homologue" references molecules having at least 60%, more preferably at least 80% and most preferably at least 90% sequence identity to one or more of the polypeptides as shown in SEQ ID NO:1, SEQ ID NO:2 or SEQ ID NO:3, or (a) fragment(s) thereof.

The invention further encompasses the determination vessel number in a patient sample, which number correlates with the tumor specific expression of one or markers of endothelial cells as known in the art, *e.g.,* the tumor specific expression level of CD31. In this context the invention encompasses the detection of homologues, variants and isoforms of one or more of endothelial cell markers or variants thereof, and may, inter alia, comprise allelic variants or splice variants of the endothelial cell markers. Also envisaged is the detection of proteins that are homologous to one or more endothelial cell markers as known in the art, *e.g.,* having at least 60%, 70%, 80%, 90%, 95%, 96%, 97%, 98% or 99% sequence identity to the amino acid sequence of a known marker for endothelial cells or a fragment thereof, *e.g.,* CD31 or a fragment thereof Alternatively or additionally, the present invention also encompasses detection of the expression levels of proteins encoded by nucleic acid sequences, or fragments thereof, which are at least at least 60%, 70%, 80%, 90%, 95%, 96%, 97%, 98% or 99% identical to a nucleic acid sequence encoding an endothelial cell marker, *e.g.,* CD31 or a fragment, variant or isoform thereof

In order to determine whether an amino acid or nucleic acid sequence has a certain degree of identity to an amino acid or nucleic acid sequence as herein described, the skilled person can use means and methods well known in the art, e.g. alignments, either manually or by using computer programs known in the art or described herein.

In accordance with the present invention, the term "identical" or "percent identity" in the context of two or more or amino acid or nucleic acid sequences, refers to two or more sequences or subsequences that are the same, or that have a specified percentage of amino acid residues or nucleotides that are the same (e.g., 60% or 65% identity, preferably, 70-95% identity, more preferably at least 95% identity with the amino acid sequences of, *e.g.,* SEQ ID NO:1, SEQ ID NO:2 or SEQ ID NO:3, or that of a known marker for endothelial cells, *e.g.,* CD31), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a defmition also applies to the complement of a test sequence. Preferably the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art. Although the FASTDB algorithm typically does not consider internal non-matching deletions or additions in sequences, *i.e.,* gaps, in its calculation, this can be corrected manually to avoid an overestimation of the % identity. CLUSTALW, however, does take sequence gaps into account in its identity calculations. Also available to those having skill in this art are the BLAST (Basic Local Alignment Search Tool) and BLAST 2.0 algorithms (Altschul, 1997, Nucl. Acids Res. 25:3389-3402; Altschul, 1993 J. Mol. Evol. 36:290-300; Altschul, 1990, J. Mol. Biol. 215:403-410). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff (1989) PNAS 89:10915) uses alignments (B) of 50, expectation (E) of 10, M=5, N=4, and a comparison of both strands.

BLAST algorithms, as discussed above, produce alignments of both amino and nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST may be used to search for identical or related molecules in protein or nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\% \text{ sequence identity } \times \% \text{ maximum BLAST score}}{100}$$

and takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, 1994, Nucl. Acids Res. 2:4673-4680) or FASTDB (Brutlag, 1990, Comp. App. Biosci. 6:237-245), as is known in the art.

The tumor specific expression levels of VEGFA, HER2 and/or neuropilin, may be considered separately, as individual markers, or in groups of two or more, as an expression profile, for the prediction of the sensitivity of a patient to the addition of bevacizumab to a chemotherapy regimen. Therefore, the methods of the invention encompass determination of an expression profile based on the expression level of one or more of the markers. As an alternative or in addition to the determination of the expression level of one or more of VEFA, HER2 and neuropilin according to the methods described herein, the vessel number in a tumor sample, relative to (a) control level(s) established in patients diagnosed with gastrointestinal cancer, in particular, mCRC, can also be used as one or more of the biomarker(s) as an indicator of a patient sensitive or responsive to the addition of angiogenesis inhibitors, *e.g.,* bevacizumab (Avastin®), to standard chemotherapies.

In accordance with the present invention, it was surprisingly discovered in the NO1966 population that a greater bevacizumab treatment effect was associated with high CD31 expression (high vessel number), higher VEGFA expression, lower neuropilin expression and lower HER2 expression on tumor cells.

The expression level of one or more of the markers VEGFA, HER2 and neuropilin may be assessed by any method known in the art suitable for determination of specific protein levels in a patient sample and is preferably determined by an immunohistochemical ("IHC") method employing antibodies specific for one or more of VEGFA, HER2, neuropilin and/or CD31. Such methods are well known and routinely implemented in the art and corresponding commercial antibodies and/or kits are readily available. For example, commercially available antibodies/test kits for VEGFA, HER2, neuropilin and CD31 can be obtained from Abcam, Inc (Cambridge, Massachusetts, U.S.A.) as clone SP28, from Dako A/S (Glostrup, Denmark) as Herceptest™, from R&D Systems, Inc. (Minneapolis, Minnesota, U.S.A.) as clone 446915, and from Dako A/S (Glostrup, Denmark) as clone JC70A, respectively. Preferably, the expression levels of the marker/indicator proteins of the invention are assessed using the reagents and/or protocol recommendations of the antibody or kit manufacturer. The skilled person will also be aware of further means for determining the expression level of one or more of VEGFA, HER2 and neuropilin by IHC methods. Therefore, the expression level of one or more of the markers/indicators of the invention can be routinely and reproducibly determined by a person skilled in the art without undue burden. However, to ensure accurate and reproducible results, the invention also encompasses the testing of patient samples in a specialized laboratory that can ensure the validation of testing procedures.

Preferably, the expression level of one or more of VEGFA, HER2 and neuropilin is assessed in biological sample that contains or is suspected to contain cancer cells. The sample may be a gastrointestinal tissue resection, a gastrointestinal tissue biopsy or a metastatic lesion obtained from a patient suffering from, suspected to suffer from or diagnosed with gastrointestinal cancer, in particular mCRC. Preferably, the sample is a sample of colorectal tissue, a resection or biopsy of a colorectal tumor, a known or suspected metastatic gastrointestinal cancer lesion or section, or a blood sample, *e.g.,* a peripheral blood sample, known or suspected to comprise circulating cancer cells, *e.g.,* gastrointestinal cancer cells. The sample may comprise both cancer cells, *i.e.,* tumor cells, and non-cancerous cells, and, in preferred embodiments, comprises both cancerous and non-cancerous cells. In aspects of the invention comprising the determination of vessel number in a sample, the sample comprises both cancer/tumor cells and non-cancerous cells that are endothelial cells. The skilled artisan, *e.g.,* a pathologist, can readily discern cancer cells from non-cancerous, *e.g.,* endothelial cells, as well as determine vessel number within a sample, *e.g.,* by staining the sample for detection of an endothelial cell marker, *e.g.,* CD31. As an alternative or additional to direct determination of vessel number, the expression level of the one or more endothelial cell markers, *e.g.,* CD31, may also be determined, which level correlates with vessel number. Methods of obtaining biological samples including tissue resections, biopsies and body fluids, *e.g.,* blood samples comprising cancer/tumor cells, are well known in the art.

In the context of the present invention, bevacizumab is to be administered in addition to or as a co-therapy or co-treatment with one or more chemotherapeutic agents administered as part of standard chemotherapy regimen as known in the art. Examples of such chemotherapeutic agents include 5-fluorouracil, leucovorin, irinotecan, gemcitabine-erlotinib, capecitabine and platinum-based chemotherapeutic agents, such as paclitaxel, carboplatin and oxaliplatin. An example of a standard chemotherapeutic regimen treatment with a combination of irinotecan, 5-fluorouracil and leucovorin, also referred to as IFL. As demonstrated in the appended examples, the addition of bevacizumab to oxaliplatin-based chemotherapeutic regimens effected an increase in progression free survival in the patients and/or patient population defined and selected according to the expression level of one or more of VEGFA, HER2, neuropilin, and CD31.

Thus the bevacizumab may be combined with an oxaliplatin-based chemotherapy regimen. Examples of oxaliplatin based chemotherapy regimens include the combination of oxaliplatin, leucovorin, and 5-fluorouracil, known as the FOLFOX4 regimen (see, *e.g.,* de Gramont et al., 2000, J. Clin. Oncol. 18:2938-2947) and the combination of oxaliplatin and capecitabine, known as the XELOX regimen (see, *e.g.,* Cassidy et al., 2004, J. Clin. Oncol. 22:2084-2091). Accordingly, in certain aspects of the invention, the patient identified according to the methods herein is treated with bevacizumab in combination with the FOLFOX4 or XELOX regimen. Common modes of administration include parenteral administration as a bolus dose or as an infusion over a set period of time, *e.g.,* administration of the total daily dose over 10 min., 20 min., 30 min., 40 min., 50 min., 60 min., 75 min., 90 min., 105 min., 120 min., 3 hr., 4 hr., 5 hr. or 6 hr. For example, 7.5 mg/kg of bevacizumab (Avastin®) may be administered to patients with colorectal cancer as an intravenous infusion over 30 to 90 minutes every three weeks as part of the XELOX regimen or at a dosage of 5 mg/kg as an intravenous infusion over 2 hours every two weeks as part of the FOLFOX4 regimen (see, *e.g.,* Saltz et al., 2008, J. Clin. Oncol. 26:2013-2019). The skilled person will recognize that further modes of administration of bevacizumab are encompassed by the invention as determined by the specific patient and chemotherapy regimen, and that the specific mode of administration and therapeutic dosage are best determined by the treating physician according to methods known in the art.

The patients selected according to the methods of the present invention are treated with bevacizumab in combination with a chemotherapy regimen, and may be further treated with one or more additional anti-cancer therapies. In certain aspects, the one or more additional anti-cancer therapy is radiation.

In preferred embodiments, the sample obtained from the patient is collected prior to beginning any other chemotherapeutic regimen or therapy, *e.g.,* therapy for the treatment of cancer or the management or amelioration of a symptom thereof Therefore, in preferred embodiments, the sample is collected before the administration of chemotherapeutics or the start of a chemotherapy regimen.

The present invention also relates to a diagnostic composition or kit comprising oligonucleotides or polypeptides suitable for the determination of expression levels of one or more of VEGFA, HER2 and neuropilin. As an alternative or additional to oligonucleotides or polypeptides suitable for the determination of expression levels of one or more of VEGFA, HER2 and neuropilin as described herein, the kit or diagnostic composition of the invention may also comprise an oligonucleotide or polypeptide for determination and/or detection of an endothelial cell marker, *e.g.,* CD31, as a means of determining vessel number as described herein. As detailed herein, oligonucleotides such as DNA, RNA or mixtures of DNA and RNA probes may be of use in detecting mRNA levels of the marker/indicator proteins, while polypeptides may be of use in directly detecting protein levels of the marker/indicator proteins via specific protein-protein interaction. In preferred aspects of the invention, the polypeptides encompassed as probes for the expression levels of one or more of VEGFA, HER2 and neuropilin (and/or one or more endothelial cell markers, *e.g.,* CD31), and included in the kits or diagnostic compositions described herein, are antibodies specific for these proteins, or specific for homologues and/or truncations thereof.

Accordingly, in a further embodiment of the present invention provides a kit useful for carrying out the methods herein described, comprising oligonucleotides or polypeptides capable of determining the expression level of one or more of VEGFA, HER2 and neuropilin (and/or one or more endothelial cell markers, *e.g.,* CD31). Preferably, the oligonucleotides comprise primers and/or probes specific for the mRNA encoding one or more of the markers/indicators described herein, and the polypeptides comprise proteins capable of specific interaction with the marker/indicator proteins, *e.g.,* marker/indicator specific antibodies or antibody fragments.

In another further embodiment, the present invention provides the use of bevacizumab for improving progression-free survival of a patient suffering from gastrointestinal cancer, in particular mCRC, comprising the following steps:

(a) obtaining a sample from said patient;
(b) determining the expression level of one or more of VEGFA, HER2 and neuropilin; and
(c) administering bevacizumab in combination with a chemotherapy regimen to the patient having an increased level of VEGFA and/or CD31, and/or a decreased level of HER2 and/or neuropilin relative to control levels determined in patients suffering from gastrointestinal cancer, in particular, mCRC.

As documented in the appended examples, the present invention solves the identified technical problem in that it could surprisingly be shown that the expression levels of one or more of VEGFA, HER2 and neuropilin in a given patient, relative to control levels determined in patients diagnosed with gastrointestinal cancer, in particular, mCRC, correlate with treatment effect in patients administered bevacizumab in combination with an oxaliplatin-based chemotherapy regimen. In the context of the invention, it was further established that a higher tumor specific vessel number, relative to control levels determined in patients diagnosed with gastrointestinal cancer, in particular, mCRC, also correlated with treatment effect in patients administered bevacizumab in combination with an oxaliplatin-based chemotherapy regimen.

The phrase "responsive to" in the context of the present invention indicates that a subject/patient suffering, suspected to suffer or prone to suffer from gastrointestinal cancer, in particular, mCRC, shows a response to a chemotherapy regimen comprising the addition of bevacizumab. A skilled person will readily be in a position to determine whether a person treated with bevacizumab according to the methods of the invention shows a response. For example, a response may be reflected by decreased suffering from gastrointestinal cancer, such as a diminished and/or halted tumor growth, reduction of the size of a tumor, and/or amelioration of one or more symptoms of gastrointestinal cancer, *e.g.,* gastrointestinal bleeding, pain, anemia. Preferably, the response may be reflected by decreased or diminished indices of the metastatic conversion of gastrointestinal cancer or indices of mCRC, *e.g.,* the prevention of the formation of metastases or a reduction of number or size of metastases,

The phrase "sensitive to" in the context of the present invention indicates that a subject/patient suffering, suspected to suffer or prone to suffer from, in particular, mCRC, shows in some way a positive reaction to treatment with bevacizumab in combination with a chemotherapy regimen. The reaction of the patient may be less pronounced when compared to a patient "responsive to" as described hereinabove. For example, the patient may experience less suffering associated with the disease, though no reduction in tumor growth or metastatic indicator may be measured, and/or the reaction of the patient to the bevacizumab in combination with the chemotherapy regimen may be only of a transient nature, *i.e.*, the growth of (a) tumor and/or (a) metastasis(es) may only be temporarily reduced or halted.

The phrase "a patient suffering from" in accordance with the invention refers to a patient showing clinical signs of gastrointestinal cancer, in particular, mCRC. The phrase "being susceptible to" or "being prone to," in the context of gastrointestinal cancer, refers to an indication disease in a patient based on, *e.g.*, a possible genetic predisposition, a pre- or eventual exposure to hazardous and/or carcinogenic compounds, or exposure to carcinogenic physical hazards, such as radiation.

The phrase "progression-free survival" in the context of the present invention refers to the length of time during and after treatment during which, according to the assessment of the treating physician or investigator, the patient's disease does not become worse, *i.e.*, does not progress. As the skilled person will appreciate, a patient's progression-free survival is improved or enhanced if the patient experiences a longer length of time during which the disease does not progress as compared to the average or mean progression free survival time of a control group of similarly situated patients.

The terms "administration" or "administering" as used herein mean the administration of an angiogenesis inhibitor, *e.g.*, bevacizumab (Avastin®), and/or a pharmaceutical composition/treatment regimen comprising an angiogenesis inhibitor, *e.g.*, bevacizumab (Avastin®), to a patient in need of such treatment or medical intervention by any suitable means known in the art for administration of a therapeutic antibody. Nonlimiting routes of administration include by oral, intravenous, intraperitoneal, subcutaneous, intramuscular, topical, intradermal, intranasal or intrabronchial administration (for example as effected by inhalation). Particularly preferred in context of this invention is parenteral administration, *e.g.*, intravenous administration. With respect to bevacizumab (Avastin®) for the treatment of colorectal cancer, the preferred dosages according to the EMEA are 5 mg/kg or 10 mg/kg of body weight given once every 2 weeks or 7.5 mg/kg or 15 mg/kg of body weight given once every 3 weeks (for details see http://www.ema.europa.eu/docs/en_GB/document_library/EPAR_-_Product_Information/human/000582/WC500029271.pdf (see page 2 bottom; formerly available under http://www.emea.europa.eu/humandocs/PDFs/EPAR/avastin/emea-combined-h582en.pdf).

The term "antibody" is herein used in the broadest sense and includes, but is not limited to, monoclonal and polyclonal antibodies, multispecific antibodies (e.g., bispecific antibodies), chimeric antibodies, CDR grafted antibodies, humanized antibodies, camelized antibodies, single chain antibodies and antibody fragments and fragment constructs, *e.g.*, F(ab')$_2$ fragments, Fab-fragments, Fv-fragments, single chain Fv-fragments (scFvs), bispecific scFvs, diabodies, single domain antibodies (dAbs) and minibodies, which exhibit the desired biological activity, in particular, specific binding to one or more of VEGFA, HER2, neuropilin and CD31, or to homologues, variants, fragments and/or isoforms thereof.

As used herein "chemotherapeutic agent" includes any active agent that can provide an anticancer therapeutic effect and may be a chemical agent or a biological agent, in particular, that are capable of interfering with cancer or tumor cells. Preferred active agents are those that act as anti-neoplastic (chemotoxic or chemostatic) agents which inhibit or prevent the development, maturation or proliferation of malignant cells. Nonlimiting examples of chemotherapeutic agents include alkylating agents such as nitrogen mustards (*e.g.*, mechlorethamine, cyclophosphamide, ifosfamide, melphalan and chlorambucil), nitrosoureas (*e.g.*, carmustine (BCNU), lomustine (CCNU), and semustine (methyl-CCNU)), ethylenimines/methylmelamines *(e.g.,* thriethylenemelamine (TEM), triethylene, thiophosphoramide (thiotepa), hexamethylmelamine (HMM, altretamine)), alkyl sulfonates *(e.g.,* busulfan), and triazines *(e.g.,* dacarbazine (DTIC)); antimetabolites such as folic acid analogs (*e.g.,* methotrexate, trimetrexate), pyrimidine analogs (*e.g.,* 5-fluorouracil, fluorodeoxyuridine, gemcitabine, cytosine arabinoside (AraC, cytarabine), 5-azacytidine, 2,2'-difluorodeoxycytidine), and purine analogs (*e.g.,* 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA)); antimitotic drugs developed from natural products (*e.g.,* paclitaxel, vinca alkaloids (*e.g.,* vinblastine (VLB), vincristine, and vinorelbine), taxotere, estramustine, and estramustine phosphate), epipodophylotoxins (*.e.g.,* etoposide, teniposide), antibiotics (*.e.g,* actimomycin D, daunomycin (rubidomycin), doxorubicin, mitoxantrone, idarubicin, bleomycins, plicamycin (mithramycin), mitomycinC, actinomycin), enzymes (*e.g.,* L-asparaginase), and biological response modifiers (*e.g.,* interferon-alpha, IL-2, G-CSF, GM-CSF); miscellaneous agents including platinum coordination complexes (*e.g.,* cisplatin, carboplatin), anthracenediones *(e.g.,* mitoxantrone), substituted urea (*i.e.,* hydroxyurea), methylhydrazine derivatives (*e.g.,* N-methylhydrazine (MIH), procarbazine), adrenocortical suppressants (*e.g.,* mitotane (o,p'-DDD), aminoglutethimide); hormones and antagonists including adrenocorticosteroid antagonists (*.e.g,* prednisone and equivalents, dexamethasone, aminoglutethimide), progestins *(e.g.,* hydroxyprogesterone caproate, medroxyprogesterone acetate, megestrol acetate), estrogens (*e.g.,* diethylstilbestrol, ethinyl estradiol and equivalents thereof); antiestrogens (*e.g.,* tamoxifen), androgens (*e.g.*, testosterone propionate, fluoxymesterone and equivalents thereof), antiandrogens (*e.g.,* flutamide, gonadotropin-releasing hormone analogs, leuprolide) and non-steroidal antiandrogens (*e.g.,* flutamide).

In the context of the present invention, "homology" with reference to an amino acid sequence is understood to refer to a sequence identity of at least 80%, particularly an identity of at least 85%, preferably at least 90% and still more preferably at least 95% over the full length of the sequence as defined by the SEQ ID NOs provided herein. In the context of this invention, a skilled person would understand that homology covers further allelic variation(s) of the marker/indicator proteins in different populations and ethnic groups.

As used herein, the term "polypeptide" relates to a peptide, a protein, an oligopeptide or a polypeptide which encompasses amino acid chains of a given length, wherein the amino acid residues are linked by covalent peptide bonds. However, peptidomimetics of such proteins/polypeptides are also encompassed by the invention wherein amino acid(s) and/or peptide bond(s) have been replaced by functional analogs, *e.g.,* an amino acid residue other than one of the 20 gene-encoded amino acids, *e.g.,* selenocysteine. Peptides, oligopeptides and proteins may be termed polypeptides. The terms polypeptide and protein are used interchangeably herein. The term polypeptide also refers to, and does not exclude, modifications of the polypeptide, *e.g.,* glycosylation, acetylation, phosphorylation and the like. Such modifications are well described in basic texts and in more detailed monographs, as well as in a voluminous research literature.

The terms "treating" and "treatment" as used herein refer to remediation of, improvement of, lessening of the severity of, or reduction in the time course of the disease or any parameter or symptom thereof. Preferably said patient is a human patient and the disease to be treated is a gastrointestinal cancer, in particular mCRC. The terms "assessing" or "assessment" of such a patient relates to methods of determining the expression levels of one or more of the marker/indicator proteins described herein, including VEGFA, HER2, neuropilin and CD31, and/or for selecting such patients based on the expression levels of such marker/indicator proteins relative to control levels established in patients diagnosed with metastatic colorectal cancer.

In addition to the methods described above, the invention also encompasses further immunohistochemical methods for assessing the expression level of one or more of VEGFA, HER2 and neuropilin, such as by Western blotting and ELISA-based detection. Similar methods may be employed in alternative or additional methods for the determination of vessel number, including the determination of tumor specific expression level of one or more endothelial cell markers, *e.g.,* CD31. As is understood in the art, the expression level of the marker/indicator proteins of the invention may also be assessed at the mRNA level by any suitable method known in the art, such as Northern blotting, real time PCR, and RT PCR. Immunohistochemical- and mRNA-based detection methods and systems are well known in the art and can be deduced from standard textbooks, such as Lottspeich (Bioanalytik, Spektrum Akademisher Verlag, 1998) or Sambrook and Russell (Molecular Cloning: A Laboratory Manual, CSH Press, Cold Spring Harbor, NY, U.S.A., 2001). The described methods are of particular use for determining the expression levels of VEGFA, HER2, neuropilin and/or CD31 in a patient or group of patients relative to control levels established in a population diagnosed with metastatic colorectal cancer.

The expression level of one or more of VEGFA, HER2 and neuropilin (and/or one or more endothelial cell markers, *e.g.,* CD31), can also be determined on the protein level by taking advantage of immunoagglutination, immunoprecipitation *(e.g.,* immunodiffusion, immunelectrophoresis, immune fixation), western blotting techniques (*e.g.,* (*in situ*) immuno histochemistry, (*in situ*) immuno cytochemistry, affinitychromatography, enzyme immunoassays), and the like. Amounts of purified polypeptide in solution may also be determined by physical methods, *e.g.* photometry. Methods of quantifying a particular polypeptide in a mixture usually rely on specific binding, *e.g.,* of antibodies. Specific detection and quantitation methods exploiting the specificity of antibodies comprise for example immunohistochemistry (*in situ*). For example, concentration/amount of marker/indicator proteins of the present invention in a cell or tissue may be determined by enzyme linked-immunosorbent assay (ELISA). Alternatively, Western Blot analysis or immunohistochemical staining can be performed. Western blotting combines separation of a mixture of proteins by electrophoresis and specific detection with antibodies. Electrophoresis may be multidimensional such as 2D electrophoresis. Usually, polypeptides are separated in 2D electrophoresis by their apparent molecular weight along one dimension and by their isoelectric point along the other direction.

As mentioned above, the expression level of the marker/indicator proteins according to the present invention may also be reflected in a decreased expression of the corresponding gene(s) encoding the VEGFA, HER2 and/or neuropilin (and/or one or more endothelial cell markers, *e.g.,* CD31, for determination of vessel number as described herein). Therefore, a quantitative assessment of the gene product prior to translation (*e.g.* spliced, unspliced or partially spliced mRNA) can be performed in order to evaluate the expression of the corresponding gene(s). The person skilled in the art is aware of standard methods to be used in this context or may deduce these methods from standard textbooks (*e.g.* Sambrook, 2001, loc. cit.). For example, quantitative data on the respective concentration/amounts of mRNA encoding one or more of VEGFA, HER2 and neuropilin (and/or one or more endothelial cell markers, *e.g.,* CD31, for determination of vessel number as described herein) can be obtained by Northern Blot, Real Time PCR and the like.

In a further aspect of the invention, the kit of the invention may advantageously be used for carrying out a method of the invention and could be, inter alia, employed in a variety of applications, e.g., in the diagnostic field or as a research tool. The parts of the kit of the invention can be packaged individually in vials or in combination in containers or multicontainer units. Manufacture of the kit follows preferably standard procedures which are known to the person skilled in the art. The kit or diagnostic compositions may be used for detection of the expression level of one or more of VEGFA, HER2 and neuropilin (and/or one or more endothelial cell markers, *e.g.,* CD31, for determination of vessel number as described herein) in accordance with the herein-described methods of the invention, employing, for example, immunohistochemical techniques described herein.

Although exemplified by the use of bevacizumab, the invention encompasses the use of other angiogenesis inhibitors as known in the art for use in combination with standard chemotherapy regimens. The terms "angiogenesis inhibitor" as used herein refers to all agents that alter angiogenesis (e.g. the process of forming blood vessels) and includes agents that block the formation of and/or halt or slow the growth of blood vessels. Nonlimiting examples of angiogenesis inhibitors include, in addition to bevacizumab, pegaptanib, sunitinib, sorafenib and vatalanib. Preferably, the angiogenesis inhibitor for use in accordance with the methods of the present invention is bevacizumab. As used herein, the term "bevacizumab" encompass all corresponding anti-VEGF antibodies or anti-VEGF antibody fragments, that fulfil the requirements necessary for obtaining a marketing authorization as an identical or biosimilar product in a country or territory selected from the group of countries consisting of the USA, Europe and Japan.

For use in the detection methods described herein, the skilled person has the ability to label the polypeptides or oligonucleotides encompassed by the present invention. As routinely practiced in the art, hybridization probes for use in detecting mRNA levels and/or antibodies or antibody fragments for use in IHC methods can be labelled and visualized according to standard methods known in the art, nonlimiting examples of commonly used systems include the use of radiolabels, enzyme labels, fluorescent tags, biotin-avidin complexes, chemiluminescence, and the like.

The person skilled in the art, for example the attending physician, is readily in a position to administer the bevacizumab in combination with a chemotherapy regimen to the patient/patient group as selected and defined herein. In certain contexts, the attending physician may modify, change or amend the administration schemes for the bevacizumab and the chemotherapy regimen in accordance with his/her professional experience. Therefore, in certain aspects of the present invention, a method is provided for the treatment or improving the progression-free survival of a patient suffering from or suspected to suffer from gastrointestinal cancer with bevacizumab in combination with a chemotherapy regimen, whereby said patient/patient group is characterized in the assessment of a biological sample (in particular a gastric tissue resection, gastric tissue biopsy or metastatic lesion), said sample exhibiting one or more of an increased expression level of VEGFA, a decreased expression level of neuropilin and a decreased expression level of HER2, relative to control levels established in patients diagnosed with metastatic colorectal cancer. The present invention also provides for the use of bevacizumab in the preparation of pharmaceutical composition for the treatment of a patient suffering from or suspected to suffer from gastrointestinal cancer, particularly mCRC, wherein the patients are selected or characterized by the herein disclosed protein marker/indicator status (*i.e.,* one or more of an increased expression level of VEGFA, a decreased expression level of neuropilin, and a decreased expression level of HER2 relative to control levels established in patients diagnosed with metastatic colorectal cancer). The invention also encompasses, alternatively or in addition the use of VEGFA, neuropilin and/or HER2 as markers as herein described, the determination of tumor specific vessel number (that may, *e.g.*, be characterized by an increased level of one or more endothelial cell markers, *e.g.*, CD31), wherein an increase in said vessel number (and/or expression level of one or more endothelial cell markers) is indicative of a patient sensitive or responsive to the addition of bevacizumab to a chemotherapeutic regimen or is selective for the patient population to which the methods herein described are directed.

The figures show:

> **Figure 1:** Forest plot of time to progression or death for bevacizumab vs. control according to tumor cell biomarker subgroup.

> **Figure 2:** Correlation of Tumor Biomarker Data with time to progression or death (median cut-off) for neuropilin (Fig. 2A), HER2 (Fig. B) and VEGFA (Fig. C). For each figure, solid black line: placebo (F/F+P/X/X+P) and biomarker expression above median (BA); long-dashed line (in grey): bevacizumab (BV) therapy (F+BV/X+BV) and biomarker expression above median (BA); medium-dashed line: bevacizumab therapy (F+BV/X+BV) and biomarker expression below median (BB); short-dashed line, placebo (F/F+P/X/X+P) and biomarker expression below median (BB).

> **Figure 3:** Forest plot of time to progression or death for bevacizumab vs. death according to endothelial biomarker subgroup.

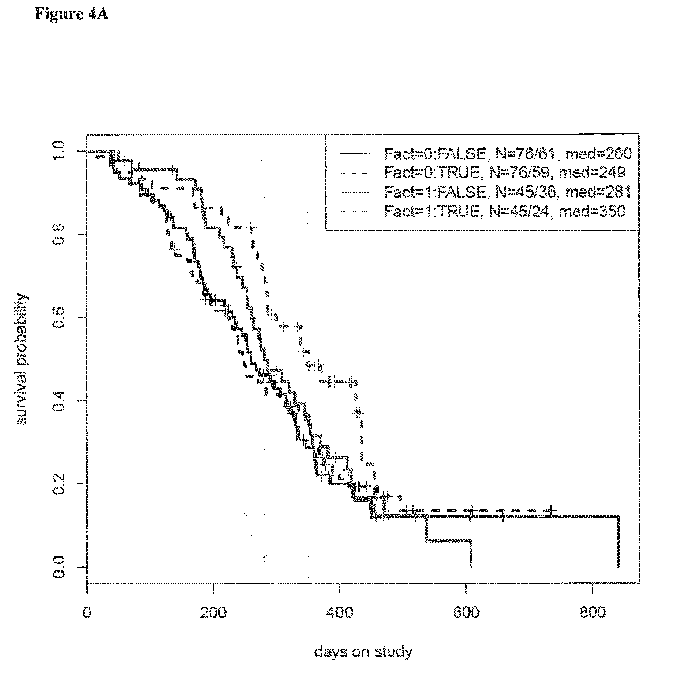

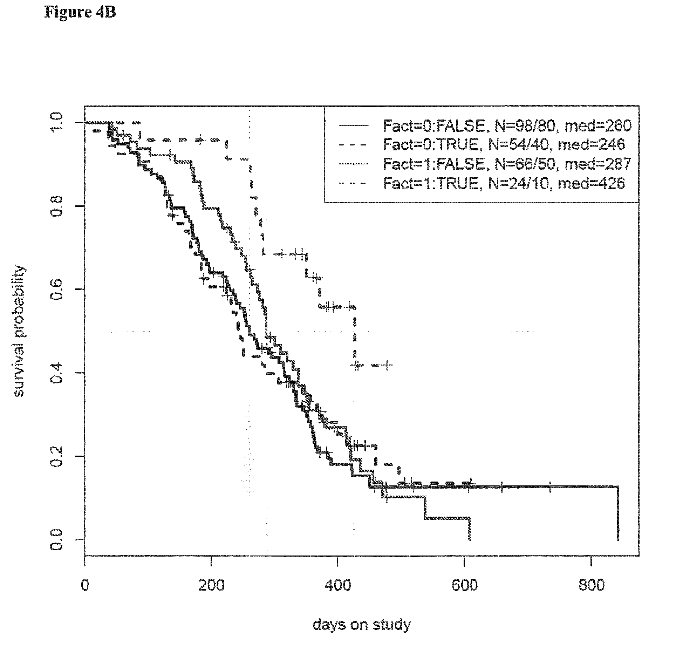

> **Figure 4:** Correlation of Endothelial Biomarker Data with time to progression or death (median cut-off) for CD31; CD31>median (Fig 4A), CD31 > $2^{nd}$ tertile (Fig. 4B). For each figure, gray solid line (ending at about day 610 on x-axis; Fact=1 in top right rectangle): bevacizumab therapy and low expression of CD31; gray dashed line (Fact=1 in top right rectangle): bevacizumab therapy and high expression of CD31; black solid line (ending at about day 85 on x-axis; Fact=0 in top right rectangle): placebo and low expression of CD31, black dashed line (levelling-off at a survival probability of about 0.14 on y-axis; Fact=0 in top right rectangle): placebo and high expression of CD31.

**Figure 5:** IHC staining of tumor and endothelial cells in representative patients with high and low microvessel densities. Nv, number of vessels; W, volume of vessels.

**Figure 6:** SEQ ID NO:1, Exemplary amino acid sequence of VEGFA.

**Figure 7:** SEQ ID NO:2, Exemplary amino acid sequence of HER2.

**Figure 8:** SEQ ID NO:3, Exemplary amino acid sequence of NRP-1.

The present invention is further illustrated by the following non-limiting examples.

**EXAMPLE 1**

Tissue samples were collected from patients participating a randomized phase-III study comparing the results of adding bevacizumab to the first-line oxaliplatin-chempterapy regimens XELOX and FOLFOX4 for the treatment of metastatic colorectal cancer (the NO 16966 study, *see,* Saltz et al., 2008, J. Clin. Oncol. 26:2013-2019 ("Saltz") and Hurwitz et al., 2004, N. Engl. J. Med. 350:2335-2342 ("Hurwitz")). An investigation of the status of biomarkers related to angiogenesis and tumorigenesis revealed that the expression levels of four biomarkers relative to control levels determined in the entire patient population correlated with an improved treatment parameter. In particular, patients exhibiting one or more of an increased expression level of VEGFA, an increased expression level of CD31, a decreased expression level HER2 and a decreased expression level of neuropilin, relative to control levels determined in the entire patient population, demonstrated a prolonged progression free survival in response to the addition of bevacizumab to either the XELOX or FOLFOX4 regimen.

**Patients and Immunohistochemical Methods**

A total of 1401 patients participated in the NO169966 study, and tumor samples from 247 of the participants were available for biomarker analysis. The baseline characteristics of the 247 patients in the biomarker analysis are provided in Table 1, which characteristics were generally similar to those of overall study population (see, Saltz; *supra*).

*Table 1. Baseline characteristics: biomarker population (n=247)*

| | FOLFOX4/XELOX + placebo | FOLFOX4/XELOX + bevacizumab |
|---|---|---|
| **Characteristic** | **(n=157)** | **(n=90)** |
| Median age, years (range) | 60(29-84) | 58.5 (18-78) |
| Male/female, n (%) | 97 (62)/60 (38) | 46 (51)/44 (49) |
| ECOG performance status, n (%) | | |
| 0 | 80(51) | 59(66) |
| 1 | 77 (49) | 30 (33) |
| 2 | 0 | 1 (1) |
| Primary tumor site, n (%) | | |
| Colon | 110 (70) | 63 (70) |
| Rectum | 31 (20) | 16 (18) |
| Colorectal | 16 (10) | 11 (12) |
| Tumor stage at diagnosis, n (%) | | |
| Local regional | 71 (45) | 27 (30) |
| Metastatic | 86 (55) | 63 (70) |
| Alkaline phosphatase, % | | |
| Normal | 39 | 38 |

(continued)

| | FOLFOX4/XELOX + placebo | FOLFOX4/XELOX + bevacizumab |
|---|---|---|
| Characteristic | (n=157) | (n=90) |
| Abnormal | 61 | 62 |
| ECOG: Eastern Cooperative Oncology Group. | | |

Immunohistochemical analysis was performed on 5 μm sections of formalin-fixed paraffin-embedded tissue samples. After deparaffinization and rehydration, antigen retrieval was performed by citrate pH 6.0 buffer at 95°C for 30 minutes in a PT module or CC1 buffer in the Benchmark-XT (Ventana, Tucson, AZ, USA).

Table 2 provides the seven markers that were selected for immunohistochemical analysis based on known tumorigenic and angiogenic activity.

*Table 2: IHC markers and antibodies used in IHC analysis*

| Target | Cell type | Staining | Clone | Distributor | Dilution | Antibody |
|---|---|---|---|---|---|---|
| CD31 | Endothelial | Membrane | JC70A | DAKO | 1/400 | mouse mAb |
| VEGF-A | Tumor | Cytoplasm | SP28 | Abeam | Prediluted | rabbit mAb |
| VEGFR-1 | Tumor and endothelial | Membrane and Cytoplasm | Y103 | Abcam | 1/30 | rabbit mAb |
| VEGFR-2 | Tumor and endothelial | Membrane | 55B11 | Cell Signaling Technology | 1/100 | rabbit mAb |
| Neuropilin | Tumor | Cytoplasm | 446915 | R&D systems | 1/75 | mouse mAb |
| EGFR | Tumor | Membrane | 2-18C9 | DAKO | | |
| HER2 | Tumor | Membrane | HercepTest | DAKO | | |

Sections were stained on Autostainer or Benchmark-XT (for VEGFR-1) and primary antibodies were incubated for 1 hour. Binding of the primary antibodies was visualized using the Envision system (DAKO, Glostrup, Denmark) or Ultraview (Ventana, Tucson, AZ USA). All sections were counterstained with Mayer's hematoxylin.

Validation reports showing accuracy, specificity, linearity, and precision (reproducibility and repeatability) are available for each IHC assay. Staining of external control slides and intrinsic control elements was documented.

**Statistical Analysis**

The overall distribution of biomarkers was described using the H-score for tumor markers. The number of markers examined was limited and each one was supported by a biological rationale; there was no formal correction for multiple testing. The a priori cut-off was used for protein expression level: median (below, above) and tertile (low, medium, high).

Treatment effects were estimated in subgroups of patients defined by biomarker level. PFS was chosen as the primary endpoint and the primary descriptive analysis was performed using subgroup analysis. Test of treatment by biomarker interactions (median cut-off) also provided a secondary analysis.

**Results**

Tumor Markers

The tumor cell associated expression of the selected IHC markers within the sample population is presented in Table 3.

*Table 3: Expression of IHC markers on samples of colorectal tumor cells*

| | | Cells with staining intensity = 0, n (%) | | |
|---|---|---|---|---|
| **Baseline biomarker** | **No. of patients** | **All cells (100%)** | **No cells (0%)** | **Some cells (>0 to <100%)** |
| VEGF-A | 241 | 4 (2) | 2 (<1) | 235 (98) |
| VEGFR-2 | 240 | 240 (100) | 0 | 0 |
| HER2 | 237 | 158 (67) | 1 (<1) | 78 (33) |
| EGFR | 240 | 88 (37) | 0 | 152 (63) |
| Neuropilin | 244 | 20 (8) | 1 (<1) | 223 (91) |
| VEGFR-1 (membrane) | 230 | 223 (97) | 0 | 7 (3) |
| VEGFR-1 (cytoplasm) | 230 | 9(4) | 5 (2) | 216 (94) |

With sole respect to tumor cells within the samples, no sample exhibited staining for VEGFR-2; however VEGFR-2 was expressed on endothelial cells. Almost no VEGFR-1 staining was observed on the tumor cell membrane; positive staining for this protein was, however, observed in the cytoplasm. Several samples also showed lack of expression of EGFR and HER2 on tumor cells: 37% of the samples showed no staining for EGFR and 67% of the samples showed no staining for HER2.

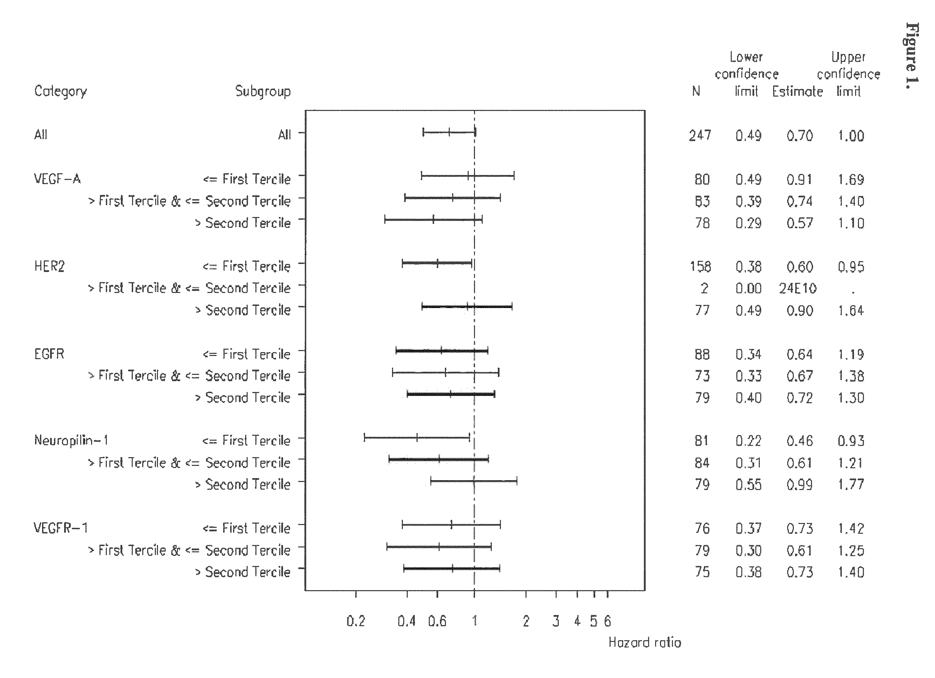

A forest plot of time to progression or death by tumor cell biomarker subgroup is shown in Figure 1. According to the hazard ratios, all patient subgroups gained benefit from bevacizumab treatment; however, patients with tumor cells having higher relative levels of VEGFA and/or low neuropilin levels showed increased benefit, while patients having HER2 positive tumors showed decreased benefit.

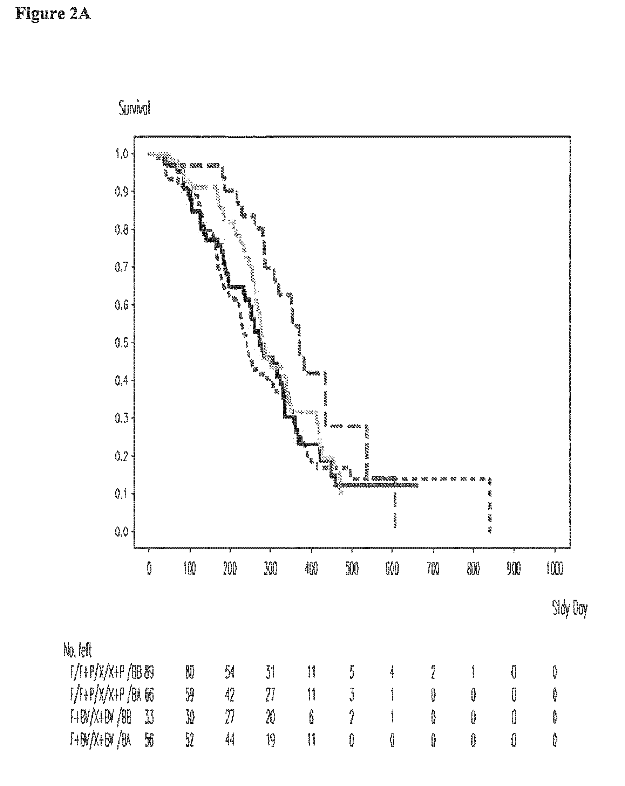

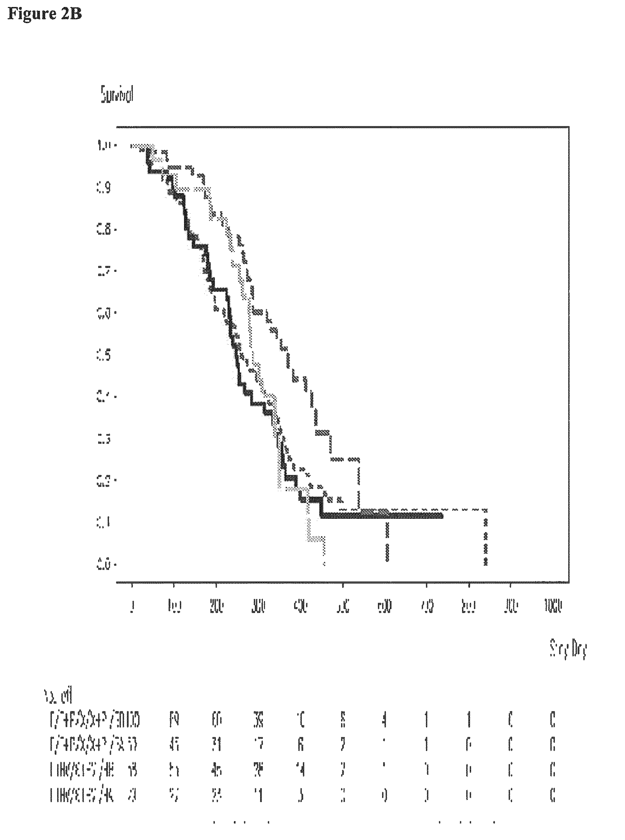

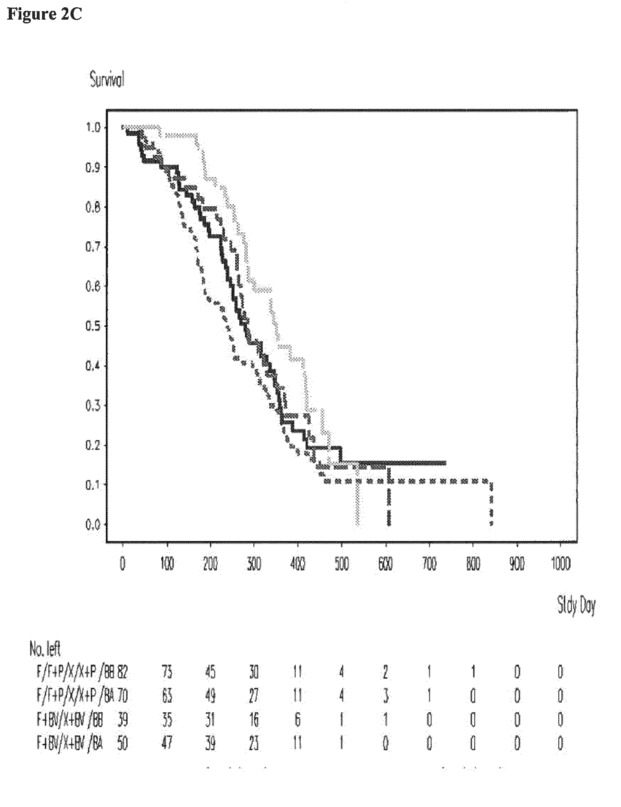

Kaplan-Meier curves for time to progression or death for these subgroups are shown in Figure 2

Endothelial Markers

The endothelial cell associated expression of the selected IHC markers within the sample population is presented in Table 4.

*Table 4: Endothelial cell biomarker IHC data: summary statistics*

| | CD31VN | | VEGFR-1VN/CD31VN | | VEGFR-2VN/CD31VN | |
|---|---|---|---|---|---|---|
| | **Placebo** | **Bevacizumab** | **Placebo** | **Bevacizumab** | **Placebo** | **Bevacizumab** |
| No. of patients | 152 | 90 | 143 | 86 | 150 | 89 |
| Mean (SD) | 72.51 (23.31) | 70.2 (19.98) | 0.06 (0.05) | 0.06 (0.06) | 0.47 (0.37) | 0.44 (0.27) |
| Coefficient of variation | 0.32 | 0.28 | 0.82 | 0.98 | 0.79 | 0.62 |
| Median (range) | 70.86 (17.17-169.67) | 71.04 (9.88-119.01) | 0.04 (0-0.21) | 0.04 (0-0.29) | 0.41 (0.01-3.39) | 0.4 (0.05-1.33) |
| 25th quartile | 58.26 | 58.27 | 0.02 | 0.02 | 0.25 | 0.26 |
| 75th quartile | 84.07 | 85.43 | 0.08 | 0.08 | 0.63 | 0.57 |

Endothelial VEGFR-1 staining was lower than endothelial VEGFR-2 staining.

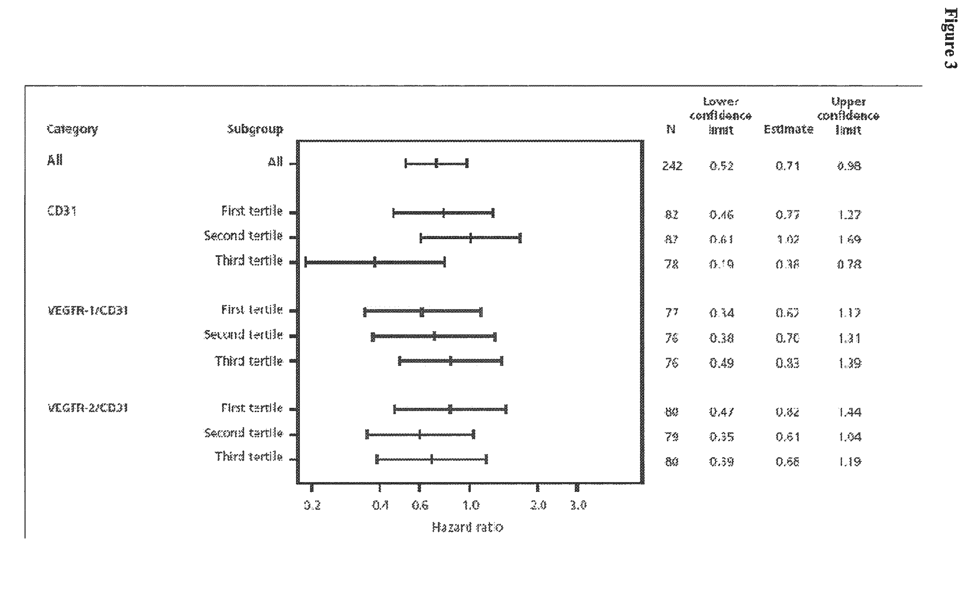

A forest plot of time to progression or death by endothelial biomarker subgroup is shown in Figure 3. Kaplan-Meier curves for time to progression or death for the CD31 subgroup are shown in Figures 4A-B. Patients having tumors exhibiting high expression of CD31, which correlates with greater numbers of blood vessels, exhibited increased benefit from bevacizumab treatment.

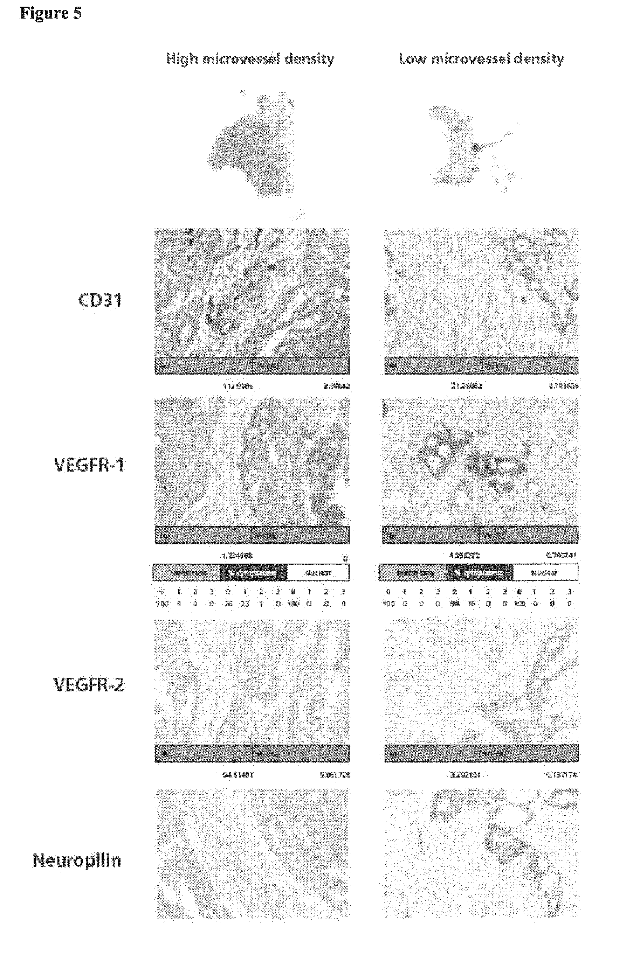

Figure 5 provides representative images of IHC samples of staining of tumor and endothelial cells in samples having high and low microvessel density.

The data provided hereinbefore were presented as Abstract No. 374 at the 2010 ASCO Gastrointestinal Cancers Symposium in Orlando Florida (January 22 to 24, 2010).

## Claims

1. An in vitro method for the identification of a patient responsive to or sensitive to the addition of bevacizumab treatment to a chemotherapy regimen, said method comprising determining the expression level of one or more of VEGFA and HER2 in a sample obtained from a patient suspected to suffer from or being prone to suffer from metastatic colorectal cancer, whereby an increased level of VEGFA and/or CD31, and/or a decreased level of HER2 relative to control levels determined in patients suffering from metastatic colorectal cancer is indicative of a sensitivity of the patient to the addition of bevacizumab to said regimen.

2. The method of claim 1, wherein the protein expression level of VEGFA is detected.

3. The method of any one of claims 1-2, wherein the protein expression level of HER2 is detected.

4. The method of any one of claims 1-3, wherein said protein expression level is detected by an immunohistochemical method (IHC).

5. The method of any one of claims 1-4, wherein said chemotherapy regimen is an oxaliplatin-based chemotherapy regimen.

6. The method of claim 5, wherein said oxaliplatin-based chemotherapy regimen is a regimen of oxaliplatin in combination with capecitabine, or a regimen of oxaliplatin in combination with leucovorin and 5-fluorouracil.

7. The method of any one of claims 1-6, wherein said patient is being co-treated with one or more anti-cancer therapies.

8. The method of claim 7, wherein said anti-cancer therapy is radiation.

9. The method of any one of claims 1-8, wherein said sample obtained from the patient is a sample obtained before neoadjuvant or adjuvant therapy.

10. Use of an oligonucleotide or polypeptide for determining the expression level of one or more of VEGFA and HER2 in the method of any one of claims 1-9.

## Figure 1.

| Category | Subgroup | N | Lower confidence limit | Estimate | Upper confidence limit |
|---|---|---|---|---|---|
| All | All | 247 | 0.49 | 0.70 | 1.00 |
| VEGF-A | <= First Tercile | 80 | 0.49 | 0.91 | 1.69 |
| | > First Tercile & <= Second Tercile | 83 | 0.39 | 0.74 | 1.40 |
| | > Second Tercile | 78 | 0.29 | 0.57 | 1.10 |
| HER2 | <= First Tercile | 158 | 0.38 | 0.60 | 0.95 |
| | > First Tercile & <= Second Tercile | 2 | 0.00 | 24E10 | . |
| | > Second Tercile | 77 | 0.49 | 0.90 | 1.64 |
| EGFR | <= First Tercile | 88 | 0.34 | 0.64 | 1.19 |
| | > First Tercile & <= Second Tercile | 73 | 0.33 | 0.67 | 1.38 |
| | > Second Tercile | 79 | 0.40 | 0.72 | 1.30 |
| Neuropilin-1 | <= First Tercile | 81 | 0.22 | 0.46 | 0.93 |
| | > First Tercile & <= Second Tercile | 84 | 0.31 | 0.61 | 1.21 |
| | > Second Tercile | 79 | 0.55 | 0.99 | 1.77 |
| VEGFR-1 | <= First Tercile | 76 | 0.37 | 0.73 | 1.42 |
| | > First Tercile & <= Second Tercile | 79 | 0.30 | 0.61 | 1.25 |
| | > Second Tercile | 75 | 0.38 | 0.73 | 1.40 |

Hazard ratio

**Figure 2A**

**Figure 2B**

**Figure 2C**

**Figure 3**

**Figure 4A**

**Figure 4B**

Figure 5

Figure 6

```
  1    MNFLLSWVHW  SLALLLYLHH  AKWSQAAPMA  EGGGQNHHEV  VKFMDVYQRS

 51    YCHPIETLVD  IFQEYPDEIE  YIFKPSCVPL  MRCGGCCNDE  GLECVPTEES

101    NITMQIMRIK  PHQGQHIGEM  SFLQHNKCEC  RPKKDRARQE  KKSVRGKGKG

151    QKRKRKKSRY  KSWSVYVGAR  CCLMPWSLPG  PHPCGPCSER  RKHLFVQDPQ

201    TCKCSCKNTD  SRCKARQLEL  NERTCRCDKP  RR    (SEQ ID NO:1)
```

## Figure 7

```
   1   MELAALCRWG LLLALLPPGA ASTQVCTGTD MKLRLPASPE THLDMLRHLY

  51   QGCQVVQGNL ELTYLPTNAS LSFLQDIQEV QGYVLIAHNQ VRQVPLQRLR

 101   IVRGTQLFED NYALAVLDNG DPLNNTTPVT GASPGGLREL QLRSLTEILK

 151   GGVLIQRNPQ LCYQDTILWK DIFHKNNQLA LTLIDTNRSR ACHPCSPMCK

 201   GSRCWGESSE DCQSLTRTVC AGGCARCKGP LPTDCCHEQC AAGCTGPKHS

 251   DCLACLHFNH SGICELHCPA LVTYNTDTFE SMPNPEGRYT FGASCVTACP

 301   YNYLSTDVGS CTLVCPLHNQ EVTAEDGTQR CEKCSKPCAR VCYGLGMEHL

 351   REVRAVTSAN IQEFAGCKKI FGSLAFLPES FDGDPASNTA PLQPEQLQVF

 401   ETLEEITGYL YISAWPDSLP DLSVFQNLQV IRGRILHNGA YSLTLQGLGI

 451   SWLGLRSLRE LGSGLALIHH NTHLCFVHTV PWDQLFRNPH QALLHTANRP

 501   EDECVGEGLA CHQLCARGHC WGPGPTQCVN CSQFLRGQEC VEECRVLQGL

 551   PREYVNARHC LPCHPECQPQ NGSVTCFGPE ADQCVACAHY KDPPFCVARC

 601   PSGVKPDLSY MPIWKFPDEE GACQPCPINC THSCVDLDDK GCPAEQRASP

 651   LTSIISAVVG ILLVVVLGVV FGILIKRRQQ KIRKYTMRRL LQETELVEPL

 701   TPSGAMPNQA QMRILKETEL RKVKVLGSGA FGTVYKGIWI PDGENVKIPV

 751   AIKVLRENTS PKANKEILDE AYVMAGVGSP YVSRLLGICL TSTVQLVTQL

 801   MPYGCLLDHV RENRGRLGSQ DLLNWCMQIA KGMSYLEDVR LVHRDLAARN

 851   VLVKSPNHVK ITDFGLARLL DIDETEYHAD GGKVPIKWMA LESILRRRFT

 901   HQSDVWSYGV TVWELMTFGA KPYDGIPARE IPDLLEKGER LPQPPICTID

 951   VYMIMVKCWM IDSECRPRFR ELVSEFSRMA RDPQRFVVIQ NEDLGPASPL

1001   DSTFYRSLLE DDDMGDLVDA EEYLVPQQGF FCPDPAPGAG GMVHHRHRSS

1051   STRSGGGDLT LGLEPSEEEA PRSPLAPSEG AGSDVFDGDL GMGAAKGLQS

1101   LPTHDPSPLQ RYSEDPTVPL PSETDGYVAP LTCSPQPEYV NQPDVRPQPP

1151   SPREGPLPAA RPAGATLERP KTLSPGKNGV VKDVFAFGGA VENPEYLTPQ

1201   GGAAPQPHPP PAFSPAFDNL YYWDQDPPER GAPPSTFKGT PTAENPEYLG

1251   LDVPV (SEQ ID NO:2)
```

**Figure 8**

```
  1   MERGLPLLCA VLALVLAPAG AFRNDKCGDT IKIESPGYLT SPGYPHSYHP

 51   SEKCEWLIQA PDPYQRIMIN FNPHFDLEDR DCKYDYVEVF DGENENGHFR

101   GKFCGKIAPP PVVSSGPFLF IKFVSDYETH GAGFSIRYEI FKRGPECSQN

151   YTTPSGVIKS PGFPEKYPNS LECTYIVFVP KMSEIILEFE SFDLEPDSNP

201   PGGMFCRYDR LEIWDGFPDV GPHIGRYCGQ KTPGRIRSSS GILSMVFYTD

251   SAIAKEGFSA NYSVLQSSVS EDFKCMEALG MESGEIHSDQ ITASSQYSTN

301   WSAERSRLNY PENGWTPGED SYREWIQVDL GLLRFVTAVG TQGAISKETK

351   KKYYVKTYKI DVSSNGEDWI TIKEGNKPVL FQGNTNPTDV VVAVFPKPLI

401   TRFVRIKPAT WETGISMRFE VYGCKITDYP CSGMLGMVSG LISDSQITSS

451   NQGDRNWMPE NIRLVTSRSG WALPPAPHSY INEWLQIDLG EEKIVRGIII

501   QGGKHRENKV FMRKFKIGYS NNGSDWKMIM DDSKRKAKSF EGNNNYDTPE

551   LRTFPALSTR FIRIYPERAT HGGLGLRMEL LGCEVEAPTA GPTTPNGNLV

601   DECDDDQANC HSGTGDDFQL TGGTTVLATE KPTVIDSTIQ SEFPTYGFNC

651   EFGWGSHKTF CHWEHDNHVQ LKWSVLTSKT GPIQDHTGDG NFIYSQADEN

701   QKGKVARLVS PVVYSQNSAH CMTFWYHMSG SHVGTLRVKL RYQKPEEYDQ

751   LVWMAIGHQG DHWKEGRVLL HKSLKLYQVI FEGEIGKGNL GGIAVDDISI

801   NNHISQEDCA KPADLDKKNP EIKIDETGST PGYEGEGEGD KNISRKPGNV

851   LKTLDPILIT IIAMSALGVL LGAVCGVVLY CACWHNGMSE RNLSALENYN

901   FELVDGVKLK KDKLNTQSTY SEA  (SEQ ID NO:3)
```

# EUROPEAN SEARCH REPORT

Europäisches
Patentamt
European
Patent Office
Office européen
des brevets

Application Number

EP 14 19 3190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YAMASHITA YORIKO ET AL: "Investigation of tumoral factors influencing the antitumor activity of bevacizumab in human gastric xenograft models", PROCEEDINGS OF THE ANNUAL MEETING OF THE AMERICAN ASSOCIATION FOR CANCER RESEARCH; 100TH ANNUAL MEETING OF THE AMERICAN-ASSOCIATION-FOR-CANCER-RESEARCH;DENVER, CA, USA; APRIL 18 20090401 AMERICAN ASSOCIATION FOR CANCER RESEARCH, US, 21 April 2009 (2009-04-21), page 1, XP008135020, ISSN: 0197-016X * abstract * | 1-10 | INV. A61K39/395 G01N33/574 |
| X | NINOMIYA S ET AL: "Effect of Bevacizumab, a Humanized Monoclonal Antibody to Vascular Endothelial Growth Factor, on Peritoneal Metastasis of MNK-45P Human Gastric Cancer in Mice", JOURNAL OF SURGICAL RESEARCH 20090615 ACADEMIC PRESS INC. USA LNKD-DOI:10.1016/J.JSS.2008.08.017, vol. 154, no. 2, 15 June 2009 (2009-06-15), pages 196-202, XP002631020, ISSN: 0022-4804 * abstract * | 1-10 | |

TECHNICAL FIELDS SEARCHED (IPC)

G01N
A61K

-/--

The present search report has been drawn up for all claims

| Place of search | Date of completion of the search | Examiner |
|---|---|---|
| The Hague | 2 March 2015 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WILLETT C G ET AL: "Efficacy, safety, and biomarkers of neoadjuvant bevacizumab, radiation therapy, and fluorouracil in rectal cancer: A multidisciplinary phase II study", JOURNAL OF CLINICAL ONCOLOGY 20090620 AMERICAN SOCIETY OF CLINICAL ONCOLOGY USA LNKD- DOI:10.1200/JCO.2008.21.1771, vol. 27, no. 18, 20 June 2009 (2009-06-20), pages 3020-3026, XP002631021, ISSN: 0732-183X * abstract * | 1-10 | |
| X | HATAKE K: "Antibody therapy in the treatment of solid tumors", BIOTHERAPY 200703 JP, vol. 21, no. 2, March 2007 (2007-03), pages 102-111, XP008175031, ISSN: 0914-2223 * abstract * | 1 | |
| A | US 2009/275057 A1 (LINKE STEVEN P [US] ET AL) 5 November 2009 (2009-11-05) * paragraphs [0021], [0042], [0097], [0098]; claims 9,11 * | 1-10 | TECHNICAL FIELDS SEARCHED (IPC) |

The present search report has been drawn up for all claims

| Place of search | Date of completion of the search | Examiner |
|---|---|---|
| The Hague | 2 March 2015 | Lunter, Pim |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

.............................................................................

& : member of the same patent family, corresponding
     document

EPO FORM 1503 03.82 (P04C01)

Europäisches
Patentamt

European
Patent Office

Office européen
des brevets

**Application Number**

EP 14 19 3190

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 14 19 3190

The Search Division considers that the present European patent application does not comply with the requirements of unity of invention and relates to several inventions or groups of inventions, namely:

1. claims: 1-10

   Method for identification of patients responsive to the addition of bevacizumab to a chemotherapy regimen; use of oligonucleotide or polypeptide; wherein the expression of VEGF-A is evaluated.

1.1. claims: 1-10

   Method for identification of patients responsive to the addition of bevacizumab to a chemotherapy regimen; use of oligonucleotide or polypeptide; wherein the expression of VEGF-A is evaluated; wherein the expression of HER2 is evaluated.

   ---

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

EP 14 19 3190

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2015

| Patent document cited in search report | Publication date | Patent family member(s) | Publication date |
|---|---|---|---|
| US 2009275057 A1 | 05-11-2009 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SALTZ et al.** *J. Clin. Oncol.,* 2008, vol. 26, 2013-2019
- **YANG et al.** *Clin. Cancer Res.,* 2008, vol. 14, 5893-5899
- **HURWITZ et al.** *N. Engl. J. Med.,* 2004, vol. 350, 2335-2342
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- **DE GRAMONT et al.** *J. Clin. Oncol.,* 2000, vol. 18, 2938-2947
- **CASSIDY et al.** *J. Clin. Oncol.,* 2004, vol. 22, 2084-2091
- **LOTTSPEICH.** Bioanalytik. Spektrum Akademisher Verlag, 1998
- **SAMBROOK ; RUSSELL.** Molecular Cloning: A Laboratory Manual. CSH Press, 2001